# EUROPÄISCHE PATENTSCHRIFT

(11) **EP 3 169 254 B1**
(45) Veröffentlichungstag und Bekanntmachung des Hinweises auf die Patenterteilung: **18.11.2020**
(21) Anmeldenummer: 15738062.7
(22) Anmeldetag: 13.07.2015
(51) Int. Cl.: A61B 17/17

(54) **POSITIONIERVORRICHTUNG ZUM FIXIEREN EINES MARKNAGELS IN EINEM RÖHRENKNOCHEN**
POSITIONING DEVICE FOR SECURING AN INTRAMEDULLARY NAIL IN A LONG BONE
DISPOSITIF DE POSITIONNEMENT POUR FIXER UN CLOU INTRAMÉDULLAIRE DANS UN OS LONG

(30) Priorität: 15.07.2014 DE 102014109935; 07.04.2015 DE 102015105242
(43) Veröffentlichungstag der Anmeldung: 24.05.2017
(73) Patentinhaber: OT Medizintechnik GmbH, 80639 München (DE)
(72) Erfinder: LUO, Hao, 80939 München (DE); SCHREIBER, Ulrich, 80639 München (DE)
(74) Vertreter: Bobbert & Partner Patentanwälte PartmbB
(86) Internationale Anmeldenummer: PCT/EP2015/065977
(87) Internationale Veröffentlichungsnummer: WO 2016/008849

(56) Entgegenhaltungen:
- EP-A1- 1 759 643
- EP-A1- 1 759 643
- EP-A1- 1 759 643
- WO-A1-2005/092219
- WO-A1-2005/092219
- WO-A1-2013/120034
- WO-A1-2013/120034
- WO-A2-2006/091625
- WO-A2-2006/091625
- DE-U1- 20 216 857
- DE-U1- 20 216 857
- DE-U1- 20 216 857
- DE-U1-202004 014 226
- DE-U1-202004 014 226
- US-A1- 2011 054 473
- US-A1- 2011 054 473
- US-A1- 2012 209 268
- US-A1- 2012 209 268
- US-A1- 2012 209 268
- US-A1- 2013 046 311
- US-A1- 2013 046 311
- US-A1- 2013 046 311

## Beschreibung

Die Erfindung bezieht sich auf eine Positioniervorrichtung zum Fixieren eines Marknagels in einem Röhrenknochen nach Anspruch 1.

Marknägel sind bekannte Hilfsmittel zur Versorgung von Brüchen langer Röhrenknochen. Sie werden in den Markraum des frakturierten Knochens eingebracht, um die Knochenfraktur mechanisch zu überbrücken. Marknägel können als sogenannte Verriegelungsmarknägel ausgestaltet sein. Bei letzteren dienen Verriegelungsschrauben dazu, die Verbindung zwischen Knochen und Verriegelungsmarknagel gegen Verschiebung zu sichern.

Bei bisher üblichen Verriegelungsmarknägeln werden die Verriegelungsschrauben in diskreten Öffnungen des Marknagels in einer vorbestimmten Stellung zum Marknagel im Knochen platziert. Das exakte Platzieren der Verriegelungsschrauben in dem im Röhrenknochen angeordneten Marknagel setzt eine große Erfahrung des Operateurs beim Einbringen des Marknagels in den Knochen voraus.

Dokument US2011/054473A1 offenbart eine exemplarische Positioniervorrichtung zum Positionieren und/oder Fixieren eines Marknagels.

Aufgabe der vorliegenden Erfindung ist es, eine Positioniervorrichtung zum Fixieren eines Marknagels in einem Röhrenknochen anzugeben.

Die erfindungsgemäße Aufgabe wird mit einer Positioniervorrichtung mit den Merkmalen des Anspruchs 1 gelöst.

Im Folgenden werden die Begriffe Verriegelungsmarknagel und Marknagel synonym verwendet.

Die erfindungsgemäße Positioniervorrichtung umfasst einen Führungsbogen mit einer Verstellvorrichtung. Die Verstellvorrichtung weist wenigstens eine Zielvorrichtung auf. Die Zielvorrichtung ist ausgebildet, um eine Verriegelungsvorrichtung, etwa eine Verriegelungsschraube, oder ein Instrument zum Einwirken auf die Verriegelungsvorrichtung, etwa einen Schraubendreher, einen Bohrer oder einen Spickdraht (Draht zur Zug-Fixierung von Knochentrümmern oder Implantaten), insbesondere lösbar, aufzunehmen.

Der Führungsbogen weist eine Hülsenführung auf, welche wiederum eine Hülse aufweist. Die Hülse ist relativ zur Hülsenaufnahme dreh- und verschiebbar in der Hülsenführung angeordnet. Hierzu weist die Hülsenaufnahme beispielsweise eine durchgängige Längsöffnung oder einen Rohrabschnitt (beide Begriffe werden hierin synonym verwendet) auf.

Die Hülse weist wiederum eine durchgängige Längsöffnung oder einen Rohrabschnitt auf, in oder an welcher/welchem optional eine Führungsvorrichtung angeordnet werden kann oder angeordnet ist.

Bei allen vorstehenden und folgenden Ausführungen ist der Gebrauch des Ausdrucks "kann sein" bzw. "kann haben" usw. synonym zu" ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

In manchen erfindungsgemäßen Ausführungsformen ist die Führungsvorrichtung vorzugsweise relativ zur Hülse verschiebbar und/oder verdrehbar in der Hülse angeordnet.

In gewissen erfindungsgemäßen Ausführungsformen ist der Führungsbogen entlang der Längsachse der Hülse relativ zur Hülse verschiebbar und um die Längsachse der Hülse relativ zu dieser drehbar angeordnet.

In manchen erfindungsgemäßen Ausführungsformen weist die Führungsvorrichtung einen Verbindungsabschnitt zum lösbaren Verbinden der Hülse mit einem Marknagel auf.

In manchen erfindungsgemäßen Ausführungsformen ist unter einem Fixieren eines Marknagels ein Verbinden des Marknagels mit Verriegelungsschrauben zu verstehen. Der Marknagel wird durch Einbringen der Verriegelungsschrauben oder -vorrichtungen im Knochen fixiert. Die erfindungsgemäße Positioniervorrichtung dient daher dem Fixieren. Sie dient auch dem Fixieren bzw. kann in manchen bestimmten Ausführungsformen hierzu jeweils verwendet werden.

Der Führungsbogen mit der Verstellvorrichtung, die eine Zielvorrichtung aufweist, kann in manchen erfindungsgemäßen Ausführungsformen als Zielbügel bezeichnet werden. Der Zielbügel ist vorzugsweise bogenförmig ausgestaltet, jedenfalls in Abschnitten hiervon.

In einigen erfindungsgemäßen Ausführungsformen ist die Verriegelungsvorrichtung eine Verriegelungsschraube oder ein Verriegelungsstift. Die vorliegende Erfindung ist hierauf allerdings nicht beschränkt.

In manchen erfindungsgemäßen Ausführungsformen ist die zur Aufnahme einer Verriegelungsschraube in dem Marknagel vorgesehene Aufnahmevorrichtung eine Vorrichtung mit wenigstens einer vorgefertigten Durchgangsöffnung für die Verriegelungsschraube. Die Aufnahmevorrichtung kann hülsenförmig oder walzenförmig ausgestaltet sein. Die Aufnahmevorrichtung kann aus mehreren Teilen und/oder mehreren Materialien zusammengesetzt sein (Composit). Beispielsweise kann ein Kunststoffring als Teil der Aufnahmevorrichtung zum Einsatz kommen. Der Kunststoffring kann ein ungewolltes Herausdrehen der Verriegelungsschraube vorteilhaft verhindern.

In gewissen erfindungsgemäßen Ausführungsformen ist die Aufnahmevorrichtung eine Öffnung oder eine Bohrung im Marknagel.

In bestimmten erfindungsgemäßen Ausführungsformen ist die Zielvorrichtung zum Setzen, Ansetzen, Führen, Ausrichten, Einbohren und/oder Einschrauben der Verriegelungsvorrichtung vorbereitet und/oder ausgestaltet.

In einigen erfindungsgemäßen Ausführungsformen ist unter der Führungsvorrichtung eine hohle oder rohrförmige oder mit einer Durchgangsöffnung in Längsrichtung versehene, vorzugsweise langgestreckte Vorrichtung zu verstehen, durch welche hindurch ein Werkzeug geführt werden kann. Treffend könnte die Führungsvorrichtung in einigen erfindungsgemäßen Ausführungsformen auch als Verspannvorrichtung oder Marknagelaufnahmevorrichtung bezeichnet werden, etwa zum Verspannen des Marknagels gegen die Zielvorrichtung, beispielsweise gegen die Hülse.

In manchen erfindungsgemäßen Ausführungsformen ist die Führungsvorrichtung eine rohrförmige, innen hohle Stange, oder ein Hohlrohr. Die Führungsvorrichtung kann zum manuellen, wieder lösbaren Fixeren oder Feststellen oder Befestigen des Marknagels an oder in der Hülse vorgesehen und vorbereitet sein.

Insbesondere kann die Führungsvorrichtung an einem axialen Ende hiervon einen Handgriff, einen Knauf oder eine ähnliche Anordnung zum manuellen Drehen der Führungsvorrichtung um ihre Längsachse, etwa beim Verschrauben der Führungsvorrichtung mit dem Marknagel, aufweisen. Der Handgriff oder Knauf kann beispielsweise gerändelt sein.

Am gegenüberliegenden Ende der Führungsvorrichtung kann diese ein Gewinde tragen, vorzugsweise ein Außengewinde, oder einen Außengewindeabschnitt. Das Außengewinde kann abgestimmt sein, um mit einem Innengewinde oder einem Innengewindeabschnitt an einem axialen Ende des Marknagels verbunden zu werden.

In gewissen erfindungsgemäßen Ausführungsformen ist die Führungsvorrichtung zum Führen eines Werkzeugs durch sie hindurch zum kraftschlüssigen Arretieren der Aufnahmevorrichtung und der in der Aufnahmevorrichtung geführten Verriegelungsvorrichtung vorgesehen und vorbereitet. Eine kraftschlüssige Arretierung kann beispielsweise mittels Festschrauben eines Gewindestifts, welcher in dem Marknagel auf die Aufnahmevorrichtung wirkt, erreicht werden.

In einigen erfindungsgemäßen Ausführungsformen ist die Führungsvorrichtung ausgestaltet, um den Marknagel lösbar gegen die Hülse zu verspannen.

In gewissen erfindungsgemäßen Ausführungsformen ist die Führungsvorrichtung koaxial zur Längsachse der Hülse angeordnet.

In einigen erfindungsgemäßen Ausführungsformen weist die Hülse, insbesondere auf einer Außen- oder Umfangsfläche hiervon, eine Kulissenführung auf, entlang welcher der Führungsbogen oder ein Abschnitt hiervon entlang der Hülse oder relativ zur Hülse bewegt werden kann.

Der Begriff "Kulissenführung", wie er hierin verwendet wird, beschreibt in manchen erfindungsgemäßen Ausführungsformen einen als Kulisse bezeichneten Schlitz, Steg, Vertiefung oder Nut, in oder auf oder entlang der eine Vorrichtung, insbesondere der Führungsbogen, geführt oder zwangsgeführt wird.

Mittels der Kulisse wird in einigen erfindungsgemäßen Ausführungsformen die Bewegung des Führungsbogens relativ zur Hülse vorgegeben oder definiert. Die Übertragungsfunktion der Kulissenführung wird durch den Verlauf des Schlitzes, des Steges, der Vertiefung oder der Nut bestimmt und vorgegeben.

Die Kulissenführung dient vorzugsweise insbesondere zum Ansteuern oder Ausrichten der Zielvorrichtung und der darin aufgenommenen Verriegelungsvorrichtung, insbesondere der Verriegelungsschraube, auf die vorgefertigten Durchgangsöffnungen des Marknagels für die Verriegelungsschraube.

Die Kulissenführung kann eine kombinierte Verschiebebewegung (in Richtung der Längsachse der Hülse) und/oder Drehbewegung (in Umfangsrichtung der Hülse) generieren.

Weiterhin kann die Kulissenführung eine Anfangsposition und/oder eine Endposition des Verschiebeweges vorgeben.

Die Kulissenführung kann als Wendelnut ausgeführt sein.

In bestimmten erfindungsgemäßen Ausführungsformen wird die Kulisse auf der Hülse mittels Erodieren, spanabhebender Bearbeitung, z. B. mittels Fräsen oder Bohren oder mittels Laserbearbeitung der Hülse hergestellt.

In gewissen erfindungsgemäßen Ausführungsformen ist die Hülse zusammen mit der Kulisse mittels eines additiven (generativen) Fertigungsverfahrens (z. B. mittels Lasersinterns) hergestellt.

In einigen erfindungsgemäßen Ausführungsformen ist die Kulisse mittels eines auftragenden Verfahrens (z. B. Schweißen) hergestellt.

In manchen erfindungsgemäßen Ausführungsformen weist die Hülse wenigstens eine Einrastposition auf, welche auf oder in der Hülse vorgesehen ist.

Die Einrastposition kann dem lösbaren Fixieren oder Arretieren des Führungsbogens in wenigstens einer vorbestimmten Position auf der Hülse oder relativ zu dieser dienen.

Die Einrastposition ist vorzugsweise nicht in die Kulissenführung integriert sondern liegt getrennt von dieser vor, beispielsweise, zumindest zum Teil, auf einer Seite der Hülse, welche jener Seite mit der Kulissenführung gegenüberliegt.

Die Einrastposition kann, beispielsweise in Umfangsrichtung der Hülse, auf einer gegenüberliegenden, um 180 Grad gedrehten Seite der Hülse, angeordnet sein.

Die Einrastposition kann eine Vertiefung in der Mantelfläche der Hülse sein. Sie kann eine Durchgangsöffnung in der Wand der Hülse sein.

Beispielsweise kann der Führungsbogen mittels eines Pins, der in den Führungsbogen integriert oder eingesteckt ist, auf der einen Seite der Hülse (in Umfangsrichtung der Hülse betrachtet) entlang der Kulissenführung geführt werden. Auf der gegenüberliegenden Seite der Hülse kann der Führungsbogen eine Einrastanordnung aufweisen, welche ausgestaltet ist, um in die Einrastposition einzugreifen, vorzugsweise um in dieser einzurasten.

Die Einrastposition kann eine Ausformung in der Kulisse sein. Sie kann ein Durchgangsloch oder eine Öffnung der Wand der Hülse sein.

Die Einrastposition kann ausgestaltet sein, um eine Arretierung des Führungsbogens an der Hülse mittels Klemmens, Rastens oder formschlüssigen Verbindens zu ermöglichen.

Der Führungsbogen kann beispielsweise mittels eines Bolzens oder Einraststifts in einer der Einrastpositionen arretiert werden. Damit wird seine Ausrichtung zur Hülse lösbar festgelegt.

In manchen erfindungsgemäßen Ausführungsformen weist der Führungsbogen wenigstens einen Einraststift oder Bolzen zum lösbaren Einrasten des Führungsbogens auf oder in der wenigstens einen Einrastposition der Hülse auf. Eine Anordnung oder Positionierung des Einraststifts in einer Einrastposition kann als Einrastanordnung bezeichnet werden. Ist der Einraststift nicht eingerastet, kann von einer Nicht-Einrastanordnung gesprochen werden. Der Einraststift kann angeordnet sein, um wiederholt zwischen den beiden vorgenannten Positionen, der Einrastanordnung und der Nicht-Einrastanordnung bewegt werden zu können.

In gewissen erfindungsgemäßen Ausführungsformen ist der Einraststift angeordnet, um manuell in die Einrastposition eingerastet oder positioniert und/oder manuell wieder ausgerastet oder entkoppelt zu werden. Für eine solche manuelle Betätigung kann eine Übersetzung, ein Getriebe, ein Schieber oder Ähnliches vorgesehen sein. Die manuelle Betätigung kann federunterstützt sein. Alternativ kann das Einrasten und/oder das Ausrasten oder Entkoppeln ohne manuelle Betätigung erfolgen, beispielsweise mittels einer federgelagerten Kugel oder einer ähnlichen Anordnung.

In einigen erfindungsgemäßen Ausführungsformen weist die Einrastposition eine in Umfangsrichtung der Hülse ausgerichtete Längsnut auf, wobei eine Längsnut eine in beliebiger Richtung verlaufende, länglich gestreckte Nut, Vertiefung oder Durchgangsöffnung in der Mantelfläche der Hülse sein kann.

Die Längsnut kann ein Verschieben des Einraststifts oder Bolzens in Umfangsrichtung der Hülse und relativ zu dieser nach dem Einrasten des Einraststifts in der Einrastposition innerhalb durch die Geometrie vorgegebene Grenzen ermöglichen. Das ermöglichte Verschieben des Einraststifts nach dem Einrasten kann als Spiel des Einraststifts in der Einrastposition bezeichnet werden. Das Spiel kann mittels der Form der Längsnut, insbesondere ihrer Länge, vorgegeben werden. Beispielsweise kann ein derartiges Spiel ein Ausrichten, Verschieben oder Positionieren der Verriegelungsschraube innerhalb einer Durchgangsöffnung für die Verriegelungsvorrichtung (z. B. einem Langloch oder einer Bohrung) im Marknagel innerhalb vorgegebener (einseitiger oder beidseitiger) Grenzen ermöglichen.

In bestimmten erfindungsgemäßen Ausführungsformen ist die Längsnut ausgeführt, um die Verriegelungsvorrichtung oder -schraube mit einem Spiel in einem definierten Maß oder Umfang, beispielsweise +/- 10° in Umfangsrichtung der Hülse, beispielsweise bezogen auf die Mitte der Durchgangsöffnung im Marknagel, zu bewegen oder zu drehen/schwenken.

In manchen erfindungsgemäßen Ausführungsformen weist der Führungsbogen eine Arretierungsvorrichtung, insbesondere eine Feststellschraube, zum vorzugsweise manuellen Arretieren des Führungsbogens in einer mittels des Einraststifts in einer ausgewählten Einrastposition vorgegebenen Stellung des Führungsbogens relativ zur Hülse auf.

Nach der Arretierung kann der Führungsbogen nicht mehr relativ zu Hülse bewegt werden.

In gewissen erfindungsgemäßen Ausführungsformen weist die Hülse Markierungen auf, mittels welcher die aktuelle Position des Führungsbogens relativ zur Hülse kontrolliert werden kann.

Die Einrastpositionen oder die Kulisse oder die Kulissenanordnung auf oder in der Hülse kann bei einem Bewegen des Führungsbogens in der Kulissenführung derart verdeckt sein, dass ein Anwender der erfindungsgemäßen Positioniervorrichtung die aktuelle oder momentane Position des Führungsbogens zwischen den Einrastpositionen oder in der Kulisse nicht sieht. Gleichwohl kann es für den Anwender hilfreich sein, beispielsweise zu erkennen, in welcher von mehreren Einrastpositionen sich der Einraststift des Führungsbogens aktuell befindet. Dies kann die weiteren Positionierungen von Verriegelungsschrauben im Marknagel mittels der erfindungsgemäßen Positioniervorrichtung vorteilhaft erleichtern und vereinfachen. Daher kann die Hülse eine Orientierungshilfe für den Anwender aufweisen, die die Kulisse und die Kulissenführung in einem sichtbaren Bereich für den Anwender auf der Oberfläche der Hülse wiedergibt oder widerspiegelt. In solchen erfindungsgemäßen Ausführungsformen kann die Kulisse in diesem sichtbaren Bereich beispielsweise mittels einer Gravur spiegelbildlich auf die Hülse aufgeprägt oder visualisiert sein.

In gewissen erfindungsgemäßen Ausführungsformen ist die Führungsvorrichtung, zum lösbaren Verbinden der Hülse mit dem Marknagel, im Inneren der Hülse und vorzugsweise koaxial oder parallel zur Längsachse der Hülse angeordnet.

Um die erfindungsgemäße Positioniervorrichtung zum Fixieren des Marknagels nutzen zu können, ist vorgesehen, die Positioniervorrichtung mit dem zu fixierenden Marknagel zu verbinden. Die hierzu vorgesehen Verbindungsanordnung weist insbesondere eine erste und eine zweite Komponente auf.

Die erste Komponente kann eine Steg-Nut-Verbindung zum verdrehsicheren gegenseitigen Anordnen von Hülse und Marknagel zueinander sein. Beispielsweise weist die Hülse an einem axialen Ende wenigstens eine, vorzugsweise aber zwei, drei oder mehr (beispielsweise axiale) Stege, Vorsprünge, Pins oder Absätze auf, die in eine entsprechende Anzahl an (beispielsweise axialen) Nuten, Schlitzen oder Vertiefungen an einem axialen Ende des Marknagels formschlüssig eingreifen. Alternativ kann der Marknagel die Absätze oder dergleichen und die Hülse die Nuten oder dergleichen aufweisen. Kombinationen hiervon sind möglich.

Nachdem die Hülse und der Marknagel derart verdrehsicher und vorzugsweise auch in vorbestimmter Weise zueinander angeordnet sind, können die Hülse und der Marknagel mittels einer zweiten Komponente miteinander verbunden werden, auf eine wieder lösbare Weise. Diese zweite Komponente kann mittels der Führungsvorrichtung realisiert werden. Die Führungsvorrichtung kann hierzu im Inneren der Hülse und koaxial zur Längsachse der Hülse angeordnet sein. Die Führungsvorrichtung kann durch die Hülse hindurch gesteckt und mittels eines am axialen Ende der Führungsvorrichtung angeordneten Außengewindes oder Außengewindeabschnitts mit einem Innengewinde oder Innengewindeabschnitt des Marknagels miteinander verschraubt werden. Die Führungsvorrichtung verspannt den Marknagel somit lösbar gegen die Hülse und hält ihn auf diese Weise mit dem Führungsbogen verbunden, vorzugsweise in vorbestimmter Ausrichtung zu Letzterem. Mittels der beiden vorstehend beschriebenen Komponenten kann vorteilhaft erreicht werden, dass sich der Marknagel, während er mit der Führungsvorrichtung verschraubt wird oder die Verschraubung mit diesem gelöst wird, also beispielsweise nach durchgeführter Fixierung des Marknagels mittels der Verriegelungsschraube(n), sich nicht in Schraubdrehrichtung dreht oder mitdreht. Ein derartiges Vermeiden einer Drehung oder Rotation, und sei diese auch nur gering, etwa in der Größenordnung von nur wenigen Grad, kann für die spätere Stabilität des Röhrensknochens vorteilhaft und wichtig sein.

In einigen erfindungsgemäßen Ausführungsformen ist die Führungsvorrichtung rohrförmig oder innen hohl und somit zum Führen oder Hindurchführen eines Werkzeugs ausgebildet. Das Werkzeug kann beispielsweise zum Betätigen oder Verschrauben (Festziehen und Lösen) einer Arretier- oder Verblockungsvorrichtung im Inneren des Marknagels dienen. Die Arretier- oder Verblockungsvorrichtung kann als Klemmschraube zum Fixieren oder Klemmen einer verstellbaren Aufnahmevorrichtung für eine Verriegelungsschraube ausgestaltet sein.

Beispielsweise kann das Werkzeug zum Verschrauben oder Festklemmen einen Innengewindestift im Marknagel aufweisen. Das Werkzeug kann ein Innensechskantschlüssel sein. Das Werkzeug kann beispielsweise zum Aufbringen eines Drehmoments von beispielsweise ca. 5 Nm, 7 Nm oder 9 Nm oder eines Bereichs zwischen 5 bis 9 Nm ausgestaltet sein.

In bestimmten erfindungsgemäßen Ausführungsformen ist die Verstellvorrichtung gemeinsam mit der auf den Marknagel auszurichtenden Zielvorrichtung beschränkt oder unbeschränkt längs des Führungsbogens verschiebbar. Die Verschieberichtung entlang des Führungsbogens wird als x-Richtung definiert. Die Zielvorrichtung ist ausgebildet, eine Verriegelungsvorrichtung, insbesondere eine Verriegelungsschraube, oder ein Instrument zum Einwirken auf die Verriegelungsvorrichtung aufzunehmen. Somit kann die Verriegelungsvorrichtung mithilfe der erfindungsgemäßen Positioniervorrichtung in einer überlagerten Bewegung entlang des Führungsbogens in x-Richtung und entsprechend der Stellung des Führungsbogens zum Marknagel entsprechend der Kulissenführung auf der Hülse ausgerichtet und positioniert werden, um nachfolgend die Verriegelungsschraube im Marknagel zu fixieren.

In manchen erfindungsgemäßen Ausführungsformen ist der Führungsbogen zumindest in Abschnitten als Kreisbogen oder kreisbogenförmig ausgebildet. Die Verstellvorrichtung ist dabei zumindest in Abschnitten längs eines Kreisbogens verschiebbar.

In gewissen erfindungsgemäßen Ausführungsformen weist die Zielvorrichtung eine oder mehrere auf den Marknagel gerichtete, durch die Verstellvorrichtung hindurch reichende oder diese durchstoßende Öffnungen auf. Die Öffnung(en) ist/sind insbesondere zur Aufnahme einer Verriegelungsvorrichtung oder eines Instruments zum Einwirken auf die in einer Aufnahmevorrichtung des Marknagels vorliegende Verriegelungsvorrichtung ausgestaltet.

In gewissen erfindungsgemäßen Ausführungsformen weist der Führungsbogen Anschläge zur Begrenzung des Verschiebewegs der Verstellvorrichtung längs des Kreisbogens auf. Die Anschläge können die Handhabung der erfindungsgemäßen Positioniervorrichtung vorteilhaft verbessern, beispielsweise indem die Verstellvorrichtung schneller oder einfacher auf eine gewünschte, ausgewählte oder anvisierte Durchgangsöffnung im Marknagel ausgerichtet werden kann. Die Anschläge können weiterhin hinsichtlich anatomisch sinnvoller Positionierungen der Verriegelungsvorrichtungen vorgesehen sein.

In einigen erfindungsgemäßen Ausführungsformen ist die Zielvorrichtung in Längsrichtung des Führungsbogens (x-Richtung) und senkrecht zur Längsrichtung des Führungsbogens (y-Richtung) in Umfangsrichtung der Hülse verschiebbar angeordnet. Mithilfe dieser Verschiebbarkeit sowohl in x-Richtung als auch in y-Richtung kann ein Instrument in der Zielvorrichtung, welches mit einer Verriegelungsvorrichtung verbunden sein kann, innerhalb eines Kreisausschnitts bewegt und positioniert werden. Die Kreisoberfläche dieses Kreisausschnitts kann durch die x-Richtung und die y-Richtung aufgespannt sein. Senkrecht zu dieser Kreisoberfläche kann die Zielvorrichtung angeordnet sein. Der Kreismittelpunkt liegt insbesondere in der Aufnahmevorrichtung für die Verriegelungsschraube im Marknagel, hier insbesondere im Schnittpunkt der Aufnahmevorrichtung der Verriegelungsschraube und der Längsachse des Marknagels (bzw. der Längsachse der Hülse). Marknagel und Positioniervorrichtung können entsprechend aufeinander abgestimmt sein. Die Bewegung des Instruments auf der Kreisoberfläche und sein Positionieren kann mit der Bewegung und Bedienung eines Joysticks verglichen werden.

In bestimmten erfindungsgemäßen Ausführungsformen ist die Zielvorrichtung in der Verstellvorrichtung und relativ zu dieser oder zum Führungsbogen verschiebbar angeordnet.

In manchen erfindungsgemäßen Ausführungsformen ist die Verstellvorrichtung in wenigstens einer ersten Position, hier als Verstellposition bezeichnet, zum Bewegen der Zielvorrichtung relativ zur Verstellvorrichtung oder zum Führungsbogen beweglich angeordnet. In wenigstens einer zweiten Position, hier als fixierte Position bezeichnet, ist die Verstellvorrichtung zum Fixieren der Zielvorrichtung relativ zur Verstellvorrichtung oder zum Führungsbogen unbeweglich angeordnet, was insbesondere mittels Reibschluss bewirkt werden kann.

Die Fixierung der Zielvorrichtung relativ zur Verstellvorrichtung oder relativ zum Führungsbogen ist vorzugsweise wieder lösbar.

Die erste Position ist insbesondere zur Positionierung und zur Ausrichtung der Verriegelungsschraube auf die gewünschte Aufnahmevorrichtung im Marknagel vorgesehen. Nachdem die Ausrichtung abgeschlossen ist und die Verriegelungsschraube anschließend in der Aufnahmevorrichtung oder durch die Aufnahmevorrichtung hindurch in dem Röhrenknochen fixiert werden soll, wird die Zielvorrichtung relativ zur Verstellvorrichtung fixiert (zweite Position). In dieser Position können die Zielvorrichtung und insbesondere die Verstellvorrichtung relativ zum Führungsbogen nicht mehr verschoben oder bewegt werden. Anschließend kann mittels eines Instruments, welches mit einer Verriegelungsschraube verbunden ist, und welches in der Zielvorrichtung angeordnet ist, die Verriegelungsschraube vorteilhaft einfach und sicher in der gewünschten Position im Marknagel und im Röhrenknochen fixiert werden.

Die Positionierung und/oder die Fixierung der Zielvorrichtung relativ zur Verstellvorrichtung können auf unterschiedliche Weise erfolgen. Den unterschiedlichen Ausführungen liegt insbesondere ein 3-Schalen-Modell oder eine 3-Schalen-Anordnung zugrunde. Die radial innere Schale kann ein Abschnitt des Führungsbogens sein. Die radial äußere Schale kann die Verstellvorrichtung oder ein Abschnitt hiervon sein. In der mittleren Schale zwischen der inneren und der äußeren Schale ist insbesondere die Zielvorrichtung integriert. Die mittlere Schale ist zwischen der äußeren und der inneren Schale bewegbar und/oder positionierbar. Die Fixierung der mittleren Schale, nachdem die Positionierung und Ausrichtung der Zielvorrichtung abgeschlossen ist, kann auf unterschiedliche Weise ausgeführt sein. Exemplarische Beispiele dieser Fixierung werden nachfolgend beschrieben.

Ein erstes Konzept zum Fixieren der mittleren Schale wird hier als Federstift-Konzept bezeichnet. Ein oder mehrere Federstifte, die beispielsweise in die äußere Schale integriert sind, drücken mit ihrer Federkraft direkt oder indirekt auf die mittlere Schale. Durch diese Anpresskräfte wird die mittlere Schale auf oder gegen die innere Schale gedrückt und mittels Reibkraft im Kontakt mit dieser fixiert. Die Anpresskräfte können anhand der Anzahl und/oder der Federstärke der Federstifte variiert werden, so dass einerseits die Zielvorrichtung bewegbar bleibt, andererseits die Reibkräfte hoch genug sind, um bei einer abgeschlossenen Positionierung und Ausrichtung eine exakte Verschraubung der Verriegelungsschraube zu ermöglichen.

Ein zweites Konzept wird hier als Klappen-Konzept bezeichnet. Die äußere Schale ist einseitig gelenkig gelagert. Auf einer (auf der Schalenoberseite) der gelenkigen Lagerung gegenüberliegenden Position ist eine Vorrichtung zum Fixieren oder Festklemmen der äußeren Schale mit der inneren Schale angeordnet. Beispielsweise kann mittels einer Flügelschraube, einem Exzenter, einem Schnapphaken oder ähnlichem die äußere Schale auf der oder gegen die mittlere und innere Schale fixiert und festgeklemmt werden. Wenn diese Vorrichtung die äußere mit der inneren Schale fixiert, z. B. durch manuelles Festziehen einer Flügelschraube, ist die mittlere Schale, in der die Zielvorrichtung angeordnet ist, festgeklemmt und unbeweglich. Wird diese Vorrichtung dagegen gelockert, kann die mittlere Schale bewegt und somit die Zielvorrichtung positioniert und ausgerichtet werden.

Ein drittes Konzept wird hier als Federn-Konzept bezeichnet. Die mittlere Schale weist zwei radial übereinander angeordnete Schalen auf. Die beiden übereinander angeordneten Schalen werden mittels Federn, die zwischen diesen beiden Schalen angeordnet sind, radial auseinander gedrückt. Weiterhin kann wenigstens eine der beiden Schalen Oberflächenstrukturen, beispielsweise Erhebungen, aufweisen, die in weitere Oberflächenstrukturen auf die radial innere Seite der äußeren, gegenüberliegenden Schale eingreifen können. Die Oberflächenstrukturen auf der Innenseite der äußeren Schale können beispielsweise Bohrungen sein, in die die Erhebungen eingreifen oder einrasten.

Ein viertes Konzept wird hier als Gewinde-Konzept bezeichnet. Die mittlere Schale weist zwei getrennt voneinander gefertigte, radial übereinander angeordnete Schalen auf, die mittels eines Gewindes miteinander verbunden sind. Durch die Drehung einer der beiden Schalen relativ zur zweiten Schale werden die Schalen, je nach Drehrichtung, entweder zusammengedreht oder auseinandergedreht, d. h. ihr Abstand zueinander verringert bzw. vergrößert sich. Werden diese beiden Schalen auseinandergedreht, werden die innere und die äußere Schale verspannt und somit fixiert.

Ein fünftes Konzept nutzt eine Hebelwirkung aus, um die äußere Schale gegen die mittlere Schale zu verspannen oder eine Verspannung zu lockern. Im Grundzustand drückt die äußere Schale im verspannten Zustand auf die mittlere Schale, die in diesem Grundzustand fixiert oder unbeweglich ist. Wenn die äußere Schale mittels Hebelwirkung nach radial außen weggedrückt oder nach außen gebogen wird, lockert sich die mittlere Schale. Die Zielvorrichtung kann ausgerichtet und positioniert werden, bis die äußere Schale wieder in den Grundzustand überführt und die mittlere Schale fixiert wird. Die Hebelwirkung wird insbesondere mittels Handkraft aufgebracht.

Manche oder alle erfindungsgemäßen Ausführungsformen können einen, mehrere oder alle der oben und/oder im Folgenden genannten Vorteile aufweisen.

Mittels der erfindungsgemäßen Positioniervorrichtung können vorteilhaft geringfügige Fehlausrichtungen beim Positionieren und/oder beim Einschrauben (dem sogenannten "Setzen") von Verriegelungsschrauben in einem im Röhrenknochen angeordneten Marknagel während einer Operation korrigiert werden.

Die Lage und der Winkel von Bohrungen für die Verriegelungsschrauben und deren Lage können mittels der erfindungsgemäßen Positioniervorrichtung vorteilhaft noch intraoperativ an die individuelle anatomische und sich aus einer Verletzung ergebende Situation angepasst werden.

Weiterhin kann vorteilhaft mittels der erfindungsgemäßen Positioniervorrichtung der Winkel der den Marknagel durchdringenden Verriegelungsschrauben noch intraoperativ variiert werden, um beispielsweise Frakturfragmente zu repositionieren oder anatomisch korrekt zu adaptieren.

Mittels der Einrastanordnung der erfindungsgemäßen Positioniervorrichtung kann durch Formschluss zwischen einerseits dem Bolzen der Einrastanordnung des Führungsbogens und andererseits der Hülse die Einrastanordnung vorteilhaft in einer definierten Position entlang der Längsachse der Hülse arretiert werden. Weiterhin kann dieser Formschluss mittels eines Langlochs in Umfangsrichtung der Hülse ausgeführt sein, um eine definierte oder begrenzende Verdrehung zwischen dem Führungsbogen und der Hülse vorzugeben oder zu ermöglichen. Dies dient dem Operateur, innerhalb bestimmter Grenzen noch intraoperativ die Verriegelungsvorrichtungen bezogen auf den Marknagel auszurichten.

Mittels der Markierungen zur Positionskontrolle des Führungsbogens relativ zur Hülse kann der Operateur vorteilhaft darin unterstützt werden, die Position von Einrastpositionen und/oder von Langlöchern entlang der Längsachse der Hülse nachzuvollziehen. Mittels der Markierungen kann die Position von Einrastpositionen und/oder von Langlöchern oder Längsnuten vorteilhaft visualisiert werden. Die Orientierung (Verdrehungsrichtung und Angabe des Winkels des Führungsbogens relativ zur Hülse) des Führungsbogens relativ zum Marknagel oder den Durchgangsöffnungen zum Einschrauben der Verriegelungsschrauben in den Marknagel kann so vorteilhaft erleichtert werden.

Erfindungsgemäß kann die Positioniervorrichtung mit dem zu fixierenden Marknagel sicher und einfach lösbar verbunden werden. Somit kann die Positioniervorrichtung vom fixierten Marknagel entkoppelt und entfernt werden. Dabei kann die erste Komponente, eine Steg-Nut-Verbindung, dem verdrehsicheren gegenseitigen Anordnen von Hülse und Marknagel dienen. Sie kann vorteilhaft sicherstellen, dass sich der Marknagel während des Verschraubens oder während des Lösens der Verschraubung zwischen Führungsvorrichtung und Marknagel, vorteilhaft nicht dreht oder mitdreht. Dies trägt dazu bei, die erzielte Stellung des Marknagels im Knochen nicht beim Verbinden der Positioniervorrichtung am Marknagel oder beim Lösen der Positioniervorrichtung vom Marknagel durch das Einleiten von Drehmoment zu gefährden.

Die vorliegende Erfindung wird im Folgenden anhand der beigefügten Figuren, in welcher identische Bezugszeichen gleiche oder ähnliche Bauteile bezeichnen, exemplarisch erläutert. In den jeweils schematisch vereinfachten Figuren gilt:
- **Fig. 1**: zeigt eine erfindungsgemäße Positioniervorrichtung einer ersten beispielhaften Ausführungsform in einer perspektivischen Ansicht;
- **Fig. 2**: zeigt die erfindungsgemäße Positioniervorrichtung aus Fig. 1 mit einem Marknagel, einer Verriegelungsschraube und einem Instrument zum Einsetzen der Verriegelungsschraube;
- **Fig. 3**: zeigt einen Abschnitt der erfindungsgemäßen Positioniervorrichtung mit einer Hülse und einer Kulissenführung in der Hülse, sowie eine Einrastanordnung;
- **Fig. 4**: zeigt den Abschnitt der erfindungsgemäßen Positioniervorrichtung aus Fig. 3 mit einem Führungsbogenabschnitt;
- **Fig. 5**: zeigt den Abschnitt der erfindungsgemäßen Positioniervorrichtung aus Fig. 3 in einer weiteren Ansicht mit mehreren Einrastpositionen und Markierungen zur Positionskontrolle;
- **Fig. 6**: zeigt die Komponenten der erfindungsgemäßen Positioniervorrichtung der Fig. 5 mit einem Führungsbogenabschnitt;
- **Fig. 7**: zeigt eine Führungsvorrichtung mit einer Hülse, verbunden mit einem Marknagel;
- **Fig. 8**: zeigt die Führungsvorrichtung aus Fig. 7, mit dem Marknagel, aber ohne die Hülse;
- **Fig. 9**: zeigt die Führungsvorrichtung aus Fig. 7;
- **Fig. 10**: zeigt eine weitere Ausführungsform der erfindungsgemäßen Positioniervorrichtung, wobei die Zielvorrichtung mittels eines Feder-Stift-Konzepts zwischen der Verstellvorrichtung und dem Führungsbogen angeordnet ist;
- **Fig. 11**: zeigt eine Detailansicht einer Verstellvorrichtung mit einer Zielvorrichtung der Positioniervorrichtung aus Fig. 10;
- **Fig. 12**: zeigt die Detailansicht der Fig. 11 ohne äußere Schale der Verstellvorrichtung;
- **Fig. 13**: zeigt eine weitere Ausführungsform der erfindungsgemäßen Positioniervorrichtung;
- **Fig. 14**: zeigt die Einrastanordnung der Ausführungsform der Fig. 13 im Detail;
- **Fig. 15**: zeigt eine weitere Einrastanordnung mit einer Spiralfeder;
- **Fig. 16**: zeigt eine weitere Einrastanordnung mit einer Blattfeder und einem Hebel;
- **Fig. 17**: zeigt eine weitere Einrastanordnung mit einem Einraststift zu dessen Einführen von schräg oben in die Hülse;
- **Fig. 18**: zeigt eine weitere Einrastanordnung mit einem Einraststift zu dessen seitlichen Einführen in die Hülse;
- **Fig. 19**: zeigt eine zweigeteilte Zielvorrichtung der Positioniervorrichtung;
- **Fig. 20**: zeigt eine Verstellvorrichtung mit einer Schnappvorrichtung zum Fixieren der Zielvorrichtung am Führungsbogen;
- **Fig. 21**: zeigt eine Zielvorrichtung mit einer Löcher-Anordnung für ein Instrument;
- **Fig. 22**: zeigt die Positioniervorrichtung der Fig. 10 in einer weiteren Ansicht;
- **Fig. 23**: zeigt die Positioniervorrichtung der Fig. 22 in einer Schnittdarstellung;
- **Fig. 24**: zeigt die Positioniervorrichtung der Fig. 22 in einer Schnittdarstellung mit einem Klappen-Konzept zum Fixieren der Verstellvorrichtung auf dem Führungsbogen;
- **Fig. 25**: zeigt eine Zielvorrichtung, welche mittels eines Federn-Konzepts zwischen der Verstellvorrichtung und dem Führungsbogen angeordnet und bewegbar ist;
- **Fig. 26**: zeigt eine Zielvorrichtung, welche mittels eines Gewinde-Konzepts zwischen der Verstellvorrichtung und dem Führungsbogen angeordnet und bewegbar ist;
- **Fig. 27**: zeigt eine weitere Ausführungsform der erfindungsgemäßen Positioniervorrichtung;
- **Fig. 28 bis 30**: zeigen eine weitere Hülse ohne Kulissenführung in verschiedenen Ansichten; und
- **Fig. 31**: zeigt einen weiteren Drehspanner.

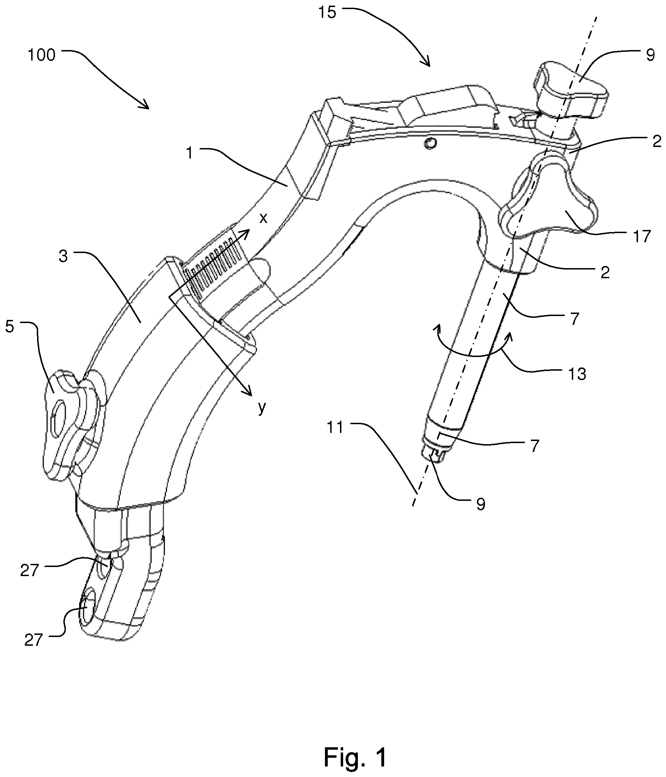

**Fig. 1** zeigt eine erfindungsgemäße Positioniervorrichtung 100 einer ersten beispielhaften Ausführungsform in einer perspektivischen Ansicht von schräg oben. Die Positioniervorrichtung 100 umfasst einen Führungsbogen 1 mit einer Hülsenführung 2, einer Verstellvorrichtung 3 und einer Zielvorrichtung 5, eine Hülse 7 sowie eine Führungsvorrichtung 9.

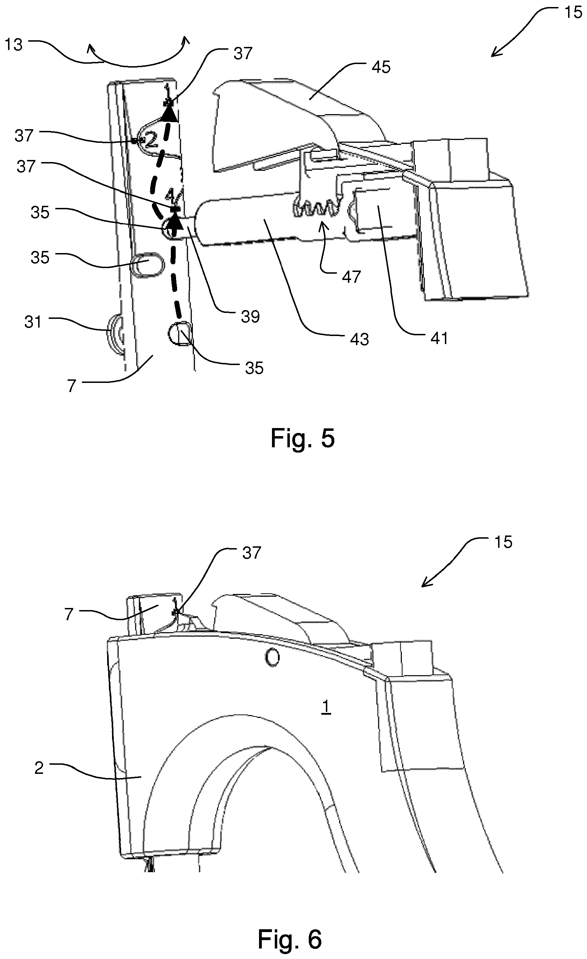

Der Führungsbogen 1 nimmt die Hülse 7 in der Hülsenaufnahme 2 auf und kann um eine Längsachse 11 der Hülse 7 relativ zur Hülse 7 verschoben und/oder um die Längsachse 11 in Umfangsrichtung 13 der Hülse 7 gedreht werden. Die Hülse 7 weist für diese Verschiebe- und Drehbewegungen des Führungsbogens 1 vorzugsweise eine Kulissenführung (oder vorgegebene Wegpfade) auf (siehe Fig. 5). Entlang dieser Kulissenführung sind optional Positionen vorgegeben (siehe Fig. 5), an denen der Führungsbogen 1 vorzugsweise mittels einer Einrastanordnung 15 eingerastet werden kann.

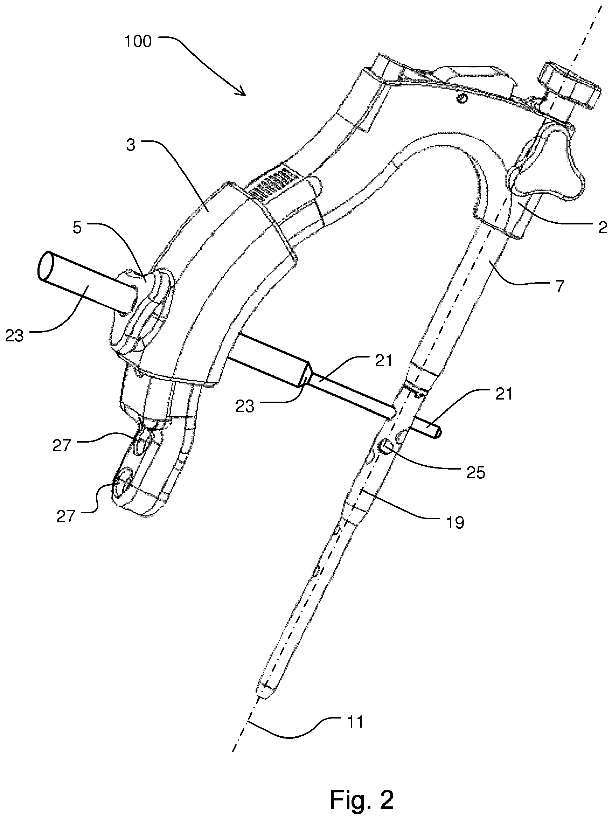

Ist, wie im Beispiel der Fig. 1 eine Einrastanordnung 15 vorgesehen, so kann in bestimmten erfindungsgemäßen Ausführungsformen nach erfolgtem Einrasten der Führungsbogen 1 mittels einer Feststellschraube 17 oder eines anderen Fixiermittels manuell auf oder an der Hülse 7 fixiert werden oder die weitere Verdrehbarkeit und/oder Längsverschiebbarkeit des Führungsbogens 1 relativ zur Hülse 7 aufgehoben werden. Nach der Fixierung ist der Führungsbogen 1 in der endgültigen Position positioniert, um anschließend einen Marknagel mittels Verriegelungsschraube zu fixieren (siehe Fig. 2).

Ist eine solche Fixierung vorgesehen, so erfolgt sie beispielsweise mittels Reibschluss oder mittels Formschluss zwischen der Feststellschraube 17 und der Hülse 7.

Die optional vorgesehene Verstellvorrichtung 3 ist entlang des Führungsbogens 1 in x-Richtung verschiebbar. Nachdem die endgültige Position der Verstellvorrichtung 3 durch Verschieben erreicht wurde, kann die Verstellvorrichtung 3 mittels der Zielvorrichtung 5, die in diesem Ausführungsbeispiel exemplarisch gleichzeitig als Feststellschraube ausgeführt ist, oder einer anders ausgestalteten Fixiervorrichtung, auf dem Führungsbogen 1 fixiert und/oder mittels Reibschluss festgeklemmt werden. Die Zielvorrichtung 5 weist hierfür vorzugsweise eine gut zu greifende Form, beispielsweise die in Fig. 1 gezeigte Dreiecksform, auf, zu ihrem manuellen Feststellen.

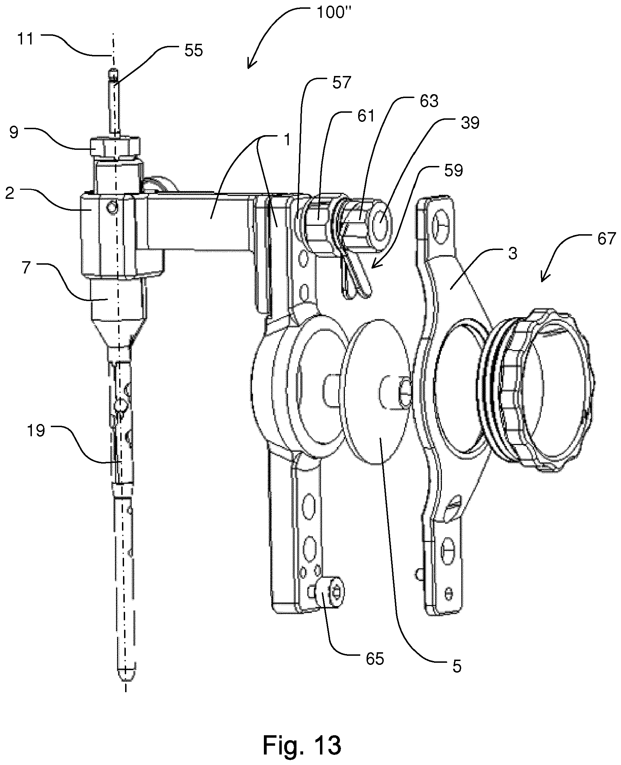

Die Führungsvorrichtung 9 hat eine in Fig. 1 nicht gezeigte Öffnung an ihrem (bezogen auf Fig. 1) oberen Ende. Sie hat ferner eine weitere Öffnung an ihrem unteren Ende, so dass sie einen durchgängigen Hohlraum zur Aufnahme eines in Fig. 1 nicht gezeigten Werkzeugs 55 (siehe Fig. 13) zum Verblocken des in Fig. 1 ebenfalls nicht gezeigten Marknagels 19 oder zur Aufnahme anderer Gegenstände bietet. Die Längsachse der Führungsvorrichtung 9 verläuft vorzugsweise parallel zu jener der Hülse 7, in welcher die Führungsvorrichtung 9 aufgenommen ist, oder ist mit der Längsachse 11 der Hülse 7 identisch.

Zusätzlich können Positionierhilfen 27 zum Positionieren von Verriegelungsschrauben an der Positioniervorrichtung 100 vorgesehen sein.

**Fig. 2** zeigt die erfindungsgemäße Positioniervorrichtung 100 aus Fig. 1 mit einem Marknagel 19, einer Verriegelungsschraube 21 und einem Instrument 23 zum Einsetzen der Verriegelungsschraube 21. Das Instrument 23 ist durch die Zielvorrichtung 5 und im vorliegenden Beispiel auch durch wenigstens einen Abschnitt des Führungsbogens 1 hindurch geführt.

Vor dem Einsetzen oder Einschrauben der Verriegelungsschraube 21 in den Marknagel 19 kann der Führungsbogen 1, wie bereits in Fig. 1 beschrieben wurde, entlang der Längsachse 11 verschoben und/oder um diese in Umfangsrichtung der Hülse 7 gedreht werden. Weiterhin kann die Verstellvorrichtung 3 entlang des Führungsbogens 1 in x-Richtung verschoben werden. Diese Positionierung (Verschiebung und Drehung) wird so lange fortgesetzt, bis eine Ausrichtung auf eine anvisierte Öffnung oder Durchgangsöffnung 25 im Marknagel 19 erzielt ist und die Verriegelungsschraube 21 im Marknagel 19 und in einem den Marknagel 19 umgebenden Röhrenknochen (in Fig. 2 nicht dargestellt) fixiert werden kann. Die erfindungsgemäße Positioniervorrichtung 100 ermöglicht vorteilhaft, diese Positionierung der Verriegelungsschraube 21 (und gegebenenfalls weiterer Verriegelungsschrauben 21) nach dem Einsetzen des Marknagels 19 in den Röhrenknochen solange fortzusetzen und dabei verschiedene Durchgangsöffnungen 25 mittels der Zielvorrichtung 5 anzusteuern, bis eine aus Sicht des Anwenders optimale Positionierung der einen oder mehreren Verriegelungsschrauben 21 erzielt wurde.

Zusätzlich können wiederum weitere Verriegelungsschrauben 21' durch die Positionierhilfen 27 hindurch oder mittels dieser in den Marknagel 19 eingeschraubt werden. Diese Positionierhilfen 27 bieten keine Möglichkeiten einer Positionierung entlang der Längsachse des Führungsbogens 1 und somit einer festen, vorgegebenen, gegebenenfalls auch senkrechten (zur Längsachse 11), oder abgewinkelten Positionierung der Verriegelungsschrauben 21 in den Marknagel. Diese Positionierhilfen 27 können als sogenannte unbewegliche Zielbohrungen für eher distale Verriegelungsschrauben 21' bezeichnet werden.

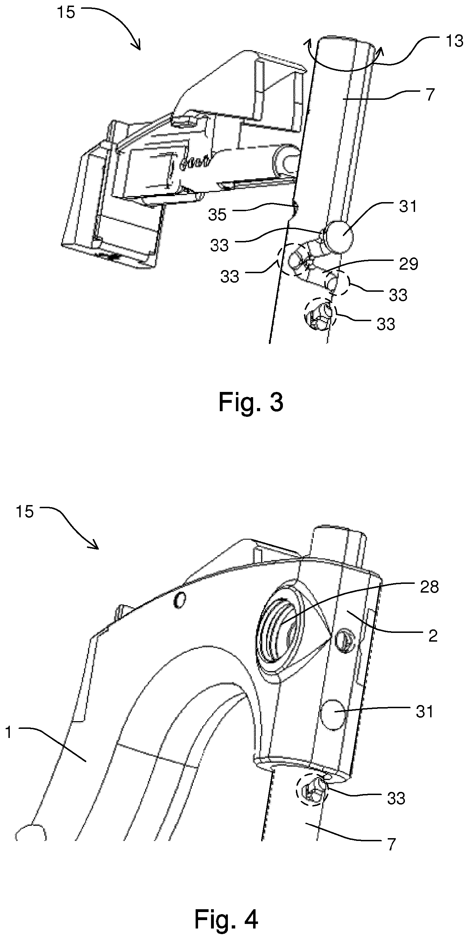

**Fig. 3** zeigt einen innen liegenden Abschnitt der erfindungsgemäßen Positioniervorrichtung 100 mit einer Hülse 7 und einer Kulissenführung 29 in oder auf der Hülse 7, sowie die Einrastanordnung 15. Eine die in Fig. 3 gezeigten Komponenten im Gebrauchszustand der Positioniervorrichtung 100 bedeckende Abdeckung, welche Teil des Führungsbogens 1 ist, ist der besseren Erkennbarkeit halber in Fig. 3 nicht dargestellt, in Fig. 4 hingegen schon.

Ein Führungsabsatz 31 (oder Pin), der in Fig. 3 vereinfacht als Einzelteil dargestellt ist, jedoch im Zusammenbau in den Führungsbogen 1 integriert ist und Teil der Hülsenführung 2 ist, erlaubt durch seinen Eingriff in die Kulissenführung 29 der Hülse 7 eine formschlüssige Verbindung zwischen dem Führungsbogen 1 bzw. der Hülsenführung 2 und der Hülse 7. Der Führungsbogen 1 wird mittels des Führungsabsatzes 31 in oder entlang der Kulissenführung 29 geführt.

Alternativ kann der Führungsabsatz 31 in die Hülse 7 integriert oder mit der Hülse 7 verbunden sein. In diesem Fall könnte die Kulissenführung 29 in die Hülsenführung 2 (s. Fig. 4) integriert sein.

Die Kulissenführung 29 ist in einem oberen Teil hiervon (bezogen auf die Darstellung der Fig. 3, also zwischen oberem Ende der Hülse 7 und dem Führungsabsatz 31) gerade ausgeführt. Mittels dieses geraden Kulissenabschnitts wird der Führungsbogen 1 beim Zusammenbau der erfindungsgemäßen Positioniervorrichtung 100 in die Hülse 7 eingeführt (oder umgekehrt).

Alternativ kann der Führungsbogen 1 auch anders mit der Hülse 7 verbunden oder auf diese montiert werden. Z. B. könnte der Außendurchmesser der Hülse 7 im oberen Bereich (oberhalb der der Kulissenführung 29) um die doppelte Tiefe der geraden Nut der Hülse kleiner gegenüber dem in Fig. 3 dargestellten Außendurchmesser sein, so dass der Führungsabsatz 31 über dem gesamten Umfang am oberen Ende der Hülse 7 auf diese aufgeschoben werden kann.

Die Kulissenführung 29 kann in einem unteren Teil hiervon als Wendelnut bezeichnet werden. Im unteren Bereich verläuft die Kulissenführung 29 nicht - oder im Wesentlichen nicht - gerade, sondern gewunden, gewendelt, geschlängelt oder dergleichen.

Der Führungsbogen 1 wird bei seinem Gebrauch daher mittels der Kulissenführung 29 entlang eines vordefinierten Weges geführt oder zumindest begrenzt. Der Führungsbogen 1 kann folglich nur entlang des durch die Kulissenführung 29 vorgezeichneten Wegs oder davon begrenzt relativ zur Hülse 7 bewegt und/oder nur in vorgegebene Positionen verschoben und/oder gedreht oder rotiert werden.

Die Führung des Führungsabsatzes 31 in der Kulissenführung 29 kann als formschlüssige Feder-Nut-Verbindung bezeichnet werden.

Entlang der Kulissenführung 29, und als Teil von dieser, sind im Beispiel der Fig. 3 an den gekrümmten oder gebogenen Stellen, Erweiterungen 33 vorgesehen. Diese können optional nutartig sein. Ihre Längsachse erstreckt sich jeweils vorzugsweise im Wesentlichen oder ausschließlich in Umfangsrichtung 13 der Hülse 7. Diese Erweiterungen 33 markieren oder codieren sogenannte Einrastpositionen 35 auf der in Umfangsrichtung 13 gegenüberliegenden Seite der Hülse 7, in welche ein weiter unten beschriebener Bolzen oder Einraststift 39 einrasten kann. Einrastpositionen 35 können als Langlöcher in Umfangsrichtung 13 bezeichnet werden. Die Einrastpositionen 35 und ihre Funktion werden zu Fig. 5 näher beschrieben. Der Aufbau und die Funktion der Einrastanordnung 15 werden ebenfalls zu Fig. 5 näher beschrieben.

**Fig. 4** zeigt den Abschnitt der erfindungsgemäßen Positioniervorrichtung 100 aus Fig. 3 eingesetzt in den oberen Abschnitt des Führungsbogens 1. In dieser Ansicht ist der Führungsabsatz 31 in den Führungsbogen 1 integriert angeordnet. Die Hülse 7 ist in den Aufnahmeabschnitt des Führungsbogens 1 für die Hülse 7, die Hülsenführung 2, aufgenommen. Die Kulissenführung 29 ist durch diesen verdeckt, lediglich die unterste, nutförmige Erweiterung 33 ist erkennbar.

Weiterhin ist in Fig. 4 ein Innengewinde 28 mit einem kegelförmigen Ansatz zur Aufnahme der Feststellschraube 17 (siehe Fig. 1) dargestellt.

**Fig. 5** zeigt den Abschnitt der erfindungsgemäßen Positioniervorrichtung 100 aus Fig. 3 in einer weiteren Ansicht. Zu erkennen sind mehrere Einrastpositionen 35 sowie Markierungen zur Positionskontrolle 37. Die Ansicht in Fig. 5 ist um ca. 180 Grad in Umfangsrichtung 13 gegenüber der Ansicht aus Fig. 3 und Fig. 4 gedreht.

Die Einrastpositionen 35 korrespondieren mit den nutförmigen Erweiterungen 33 der Kulissenführung 29 (siehe Fig. 3) sowie den Markierungen zur Positionskontrolle 37 ("1", "2" und "4"; die Position "3" ist verdeckt und nicht sichtbar). Beispielsweise korrespondiert die Markierung "1" mit der obersten Einrastposition, die in Fig. 5 mit dem Bolzen 39 eingerastet ist. Diese Anordnung korrespondiert mit der Anordnung in Fig. 3 und Fig. 4, in welcher der Führungsabsatz 31 in der obersten nutförmigen Erweiterung 33 der Kulissenführung 29 dargestellt ist.

Eine Arretierung der Einrastanordnung 15, und damit des Führungsbogens 1, in den die Einrastanordnung 15 integriert ist und welcher mittels der Arretierung in Bezug auf die Hülse 7 festgelegt bzw. in weiterer Bewegung oder Rotation relativ zur Hülse 7 beschränkt wird, erfolgt mittels Formschluss zwischen dem Bolzen 39 und der Einrastposition 35 an einer zuvor festgelegten und definierten Position der Hülse 7. Bevor der Bolzen 39 in einer Einrastposition 35 einrastet, kann der Bolzen 39 mittels einer Vorspannung, insbesondere mittels einer Feder erzielt, auf die Hülse 7 einwirken. Der Bolzen 39 wird dann in Kontakt (reibend) entlang der Hülse 7 geführt.

Die Einrastposition 35 ist als Langloch ausgeführt, kann jedoch auch andere beliebige Formen aufweisen. Bei einer Langlochform der Einrastposition 35 kann sich der Führungsbogen 1 innerhalb des Langlochs in Umfangsrichtung 13 der Hülse 7 bewegen. Dieses sogenannte Spiel des Führungsbogens 1 in der Hülse 7 kann die Positionierung und Verschraubung der Verriegelungsschraube 21 in dem Marknagel 19 erleichtern (siehe Fig. 2).

Die Markierungen zur Positionskontrolle 37 ("1", "2" und "4") sind Visualisierungshilfen und damit Orientierungshilfen für den Anwender der Positioniervorrichtung 100 bezüglich der Verdrehungsrichtung und/oder der Angabe eines Winkels des Führungsbogens 1. Mithilfe dieser Markierungen 37 kann der Anwender die Lage der Einrastpositionen 35 einfach nachvollziehen.

Die Arretierung des Bolzens 39 in einer der Einrastpositionen 35 erfolgt mittels der Einrastanordnung 15. Im eingerasteten Zustand, in welchem der Bolzen 39 in der Einrastposition 35 steckt, drückt vorzugweise eine Spannvorrichtung, etwa eine doppelte Blattfeder 41 wie jene der Fig. 5, auf die Bolzenanordnung 43 (entspricht einer Verlängerung des Bolzens 39) und verspannt diese in der Einrastposition 35. Ein ungewolltes Herausrutschen des Bolzens 39 aus der Einrastposition 35 kann hierdurch vorteilhaft verhindert werden. Eine Entkoppelung der Einrastposition 35 erfolgt mittels, insbesondere manuellen, Niederdrückens eines Hebels 45 (oder durch Betätigen einer anderen, geeigneten Vorrichtung), der beispielsweise mittels einer Zahnradverbindung 47 die Bolzenanordnung 43, und damit den Bolzen 39, herauszieht oder entkoppelt. Im Anschluss an die Entkopplung kann der Führungsbogen 1, relativ zur Hülse 7, erneut gemäß dem Verlauf der Kulissenführung bewegt und beispielsweise in einer weiteren Einrastposititon 35 positioniert werden.

Fig. 6 zeigt die Komponenten der erfindungsgemäßen Positioniervorrichtung 100, welche in Fig. 5 zu sehen sind, zumindest bedeckt durch einen Abschnitt des Führungsbogens 1, der wie bereits zu Fig. 4 diskutiert die Einrastanordnung 15 verdeckt.

Der Führungsbogen 1 verdeckt im vorliegenden Beispiel alle vier Einrastpositionen 35, so dass der Anwender zunächst nicht erkennen kann, in welcher Einrastposition 35 der Bolzen 39 eingerastet ist. Aus diesem Grund ist optional die Markierung 37 zur Positionskontrolle an der Oberfläche der Hülse 7 vorgesehen. In Fig. 6 ist erkennbar, dass sich der Bolzen 39 in der obersten Einrastposition 35 befindet, da die Markierung 37 auf die "1", also die oberste Markierung 37, vergleiche mit Fig. 5, zeigt.

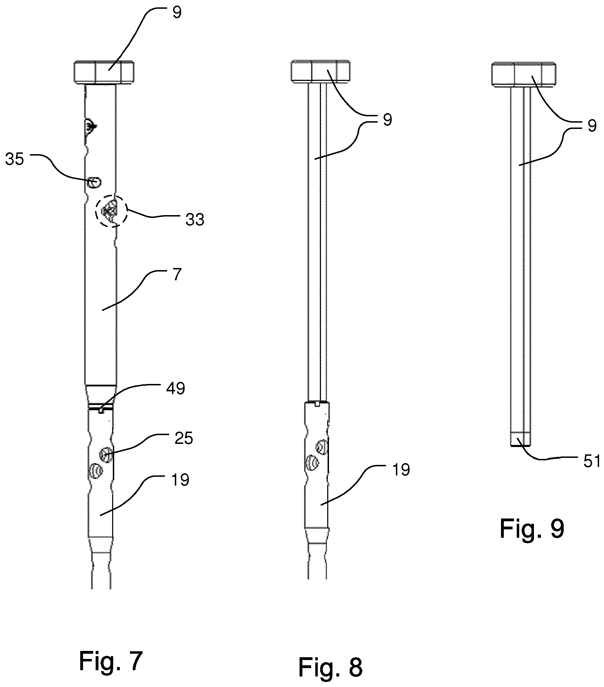

**Fig. 7** zeigt die Führungsvorrichtung 9, eingesteckt in die eine Längsöffnung aufweisende Hülse 7, und verbunden mit dem Marknagel 19.

Die Hülse 7 ist exemplarisch mittels zweier Stege 49 (in Fig. 7 ist nur der vordere Steg 49 sichtbar) verdrehsicher mit dem Marknagel 19 verbunden, wobei die Stege 49 formschlüssig in Nuten des Marknagels 19 eingeführt sind. Diese Verbindung kann als Feder-Nut-Verbindung bezeichnet werden. Zum Fixieren der Hülse 7 mit dem Marknagel 19 wird beispielsweise ferner ein optionales Gewinde 51 (Außengewinde) am unteren Ende der Führungsvorrichtung 9 (siehe Fig. 9) in ein Innengwinde des Marknagels 19, falls vorhanden, eingeschraubt. Der Marknagel 19 ist somit verdreh- wie verschiebesicher an der Hülse 7 festgelegt ("adaptiert"). Die Lage oder Position der Hülse 7 relativ zum Marknagel 19 ist somit vorzugsweise sowohl in Längsrichtung als auch in Umfangsrichtung festgelegt.

**Fig. 8** zeigt die Führungsvorrichtung 9 aus Fig. 7 mit nur dem Marknagel 19, ohne Hülse 7.

**Fig. 9** zeigt die Führungsvorrichtung 9 aus Fig. 7 als Einzelteil mit dem Außengewinde 51 am unteren Ende der Führungsvorrichtung 9.

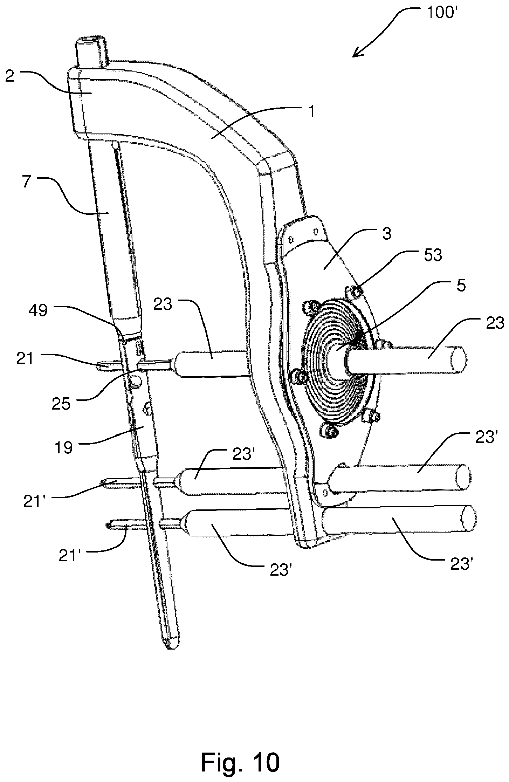

**Fig. 10** zeigt eine weitere Ausführungsform einer erfindungsgemäßen Positioniervorrichtung 100'.

Die Verstellvorrichtung 3 ist als Abschnitt einer Kugeloberfläche mit einer kreisrunden Öffnung ausgeführt. Die Form der Verstellvorrichtung 3 kann anstatt einer kugelförmigen Oberfläche gleichfalls eine anders gestaltete, nur optional gekrümmte oder gerade Oberfläche aufweisen.

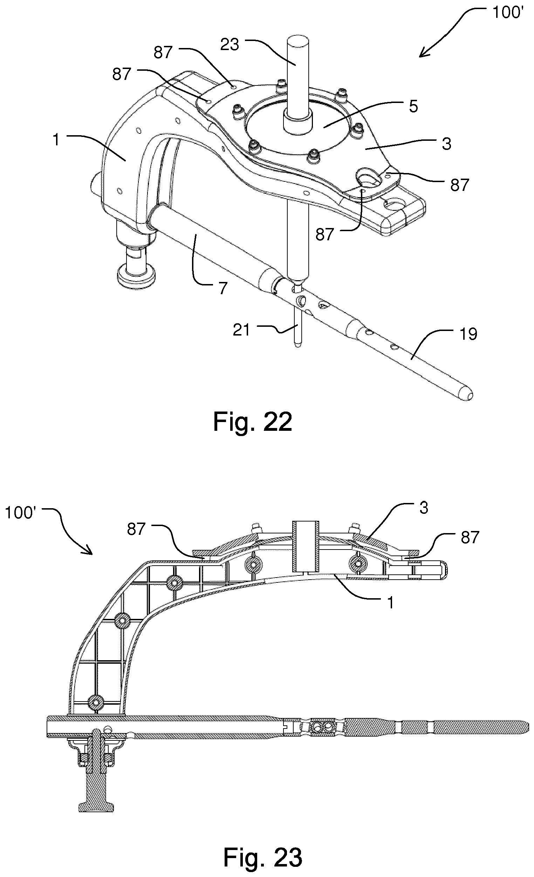

Die Verstellvorrichtung 3 ist auf dem Führungsbogen 1 fixiert (die nähere Beschreibung der Fixierung wird zu den Fig. 22 und 23 erläutert).

Die beispielsweise kreisrunde Öffnung in einem zentralen oder mittleren Bereich der Verstellvorrichtung 3 ist zur Führung der Zielvorrichtung 5 und des Instruments 23 (welches zum Einsetzen der Verriegelungsschraube 21 dient) vorgesehen. Die Längsachse des Instruments 23 steht senkrecht zur Kugeloberfläche. Die Verlängerung der Längsachse des Instruments 23 verläuft durch den Mittelpunkt der Bohrung oder Durchgangsöffnung im Marknagel 19.

Das Instrument 23 wird in der Zielvorrichtung 5 geführt. Die Zielvorrichtung 5 ist optional schalenförmig mit ringförmig konzentrisch angeordneten Nuten, Rillen oder Erhebungen um die mittlere Führung des Instruments 23 herum ausgeführt. Die schalenförmige Zielvorrichtung 5 kann als Mittelschale bezeichnet werden, die, in radialer Richtung betrachtet, zwischen der äußeren Schale, welche der Verstellvorrichtung 3 entspricht, und der inneren Schale, welche der schalenförmig anliegenden Oberfläche der Führungsbogens 1 entspricht, angeordnet ist.

Die Zielvorrichtung 5, also die mittlere Schale, kann zwischen der inneren und der äußeren Schale bewegt oder verschoben werden. Bei dieser Bewegung greifen optionale, in Radialrichtung federnd gelagerte Stifte 53 oder Pins in die ringförmigen Nuten oder Rillen der Zielvorrichtung 5 ein. Damit wird eine definierte und exakte Positionierung des Instruments 23, und damit der Verriegelungsschraube 21, möglich. Die Nutabstände zueinander entsprechen einer bestimmten und vorgegebenen Auslenkung der Zielvorrichtung 5, die in einem Winkel oder einer Gradzahl angegeben werden kann. In diesem Ausführungsbeispiel entspricht die Auslenkung zwischen zwei ringförmigen Nuten einem Winkel oder einer Gradzahl von einem Grad (1°). Dieses Prinzip oder Konzept mit federnd gelagerten Stiften 53, die in Nuten eingreifen, kann als Feder-Stift-Konzept bezeichnet werden.

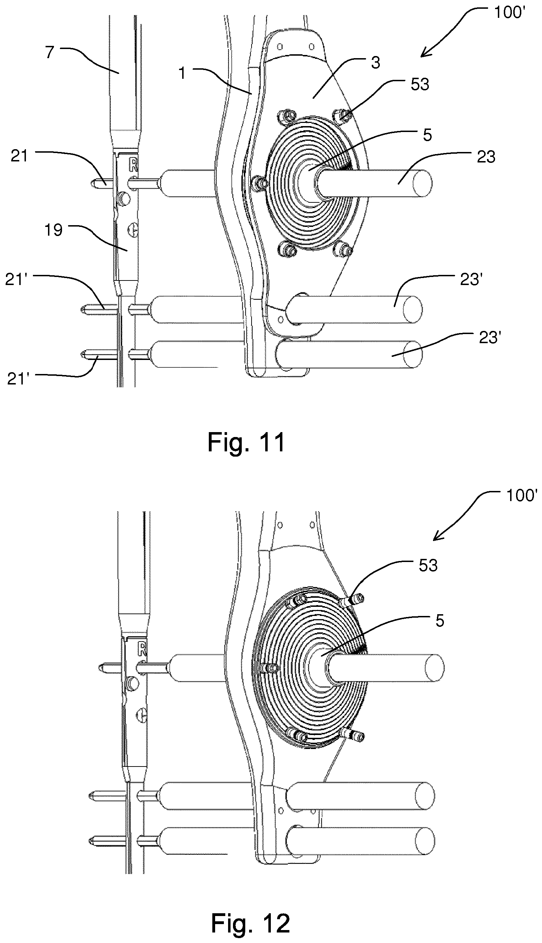

Die Anordnung der in Radialrichtung federnd gelagerten Stifte 53 auf der radial äußeren Oberfläche der Zielvorrichtung 5 wird in Fig. 12 gezeigt, in der die Verstellvorrichtung 3 (äußere Schale) zur besseren Anschaulichkeit entfernt wurde.

Die Anordnung in Fig. 10 ermöglicht eine Auslenkung des Instruments 23 (und damit der Verriegelungsschraube 21) von einer gewünschten oder vorgegebenen Gradzahl (oder einem Gradbereich), beispielsweise von zehn Grad (10°) gegen über der zentrischen Ausgangsposition in der Mitte (diese Ausgangsposition wird in den Fig. 10, 11 und 12 gezeigt und als Null-Grad-Position (0°) bezeichnet).

Mittels eines optionalen, kombinierten Formschlusses (zwischen den Stiften 53 und den ringförmigen Nuten der Zielvorrichtung 5) und eines Reibschlusses (zwischen der mittleren und der äußeren Schale) wird ein Fixieren der Position des Instrumentes 23 zum Einschrauben oder Setzen der Verriegelungsschraube 21 ermöglicht.

Die Zielvorrichtung 5 kann optional einem kreisrunden Abschnitt einer Struktur aufliegen, welche unter der Zielvorrichtung 5 angeordnet ist. Auf diese Weise kann sichergestellt werden, dass die Zielvorrichtung 5 vorzugsweise unter gleichbleibendem Abstand zu einem Zielpunkt geführt wird.

**Fig. 11** zeigt eine Detailansicht aus Fig. 10 mit der Verstellvorrichtung 3 (äußere Schale), der Zielvorrichtung 5 (mittlere Schale), dem Führungsbogen 1 (innere Schale), der Hülse 7, dem Marknagel 19 und dem Instrument 23 zum Einsetzen der Verriegelungsschraube 21. Weiterhin werden nicht verschiebbare Instrumente 23' (die Zielvorrichtung 3 ist relativ zum Führungsbogen 1 nicht verschiebbar) zum Verschrauben oder Einschrauben von weiteren Verriegelungsschrauben 21' dargestellt.

**Fig. 12** zeigt die Ansicht aus Fig. 11 ohne die Verstellvorrichtung 3 (äußere Schale) zum Darstellen der Position der Stifte 53 auf der Zielvorrichtung 5 (mittlere Schale).

**Fig. 13** zeigt eine weitere Ausführungsform der erfindungsgemäßen Positioniervorrichtung 100".

Der Führungsbogen 1 ist in dieser Ausführungsform zweigeteilt. Ein erster Abschnitt (in Fig. 13 der linke Abschnitt) des zweigeteilten Führungsbogens 1 weist die Hülsenführung 2 auf, ist exemplarisch gerade ausgeführt und verläuft in diesem Ausführungsbeispiel im Wesentlichen senkrecht zur Längsachse 11 der Hülse 7. In die Hülse 7 ist die Führungsvorrichtung 9 eingeführt, in der wiederum das Werkzeug 55, beispielsweise ein Sechskantdreher, beispielsweise zum Verblocken des Marknagels 19, entlang der Längsachse 11 geführt wird. Alternativ können anstatt des Werkzeugs 55 auch andere Gegenstände mittels der Führungsvorrichtung 9 geführt werden.

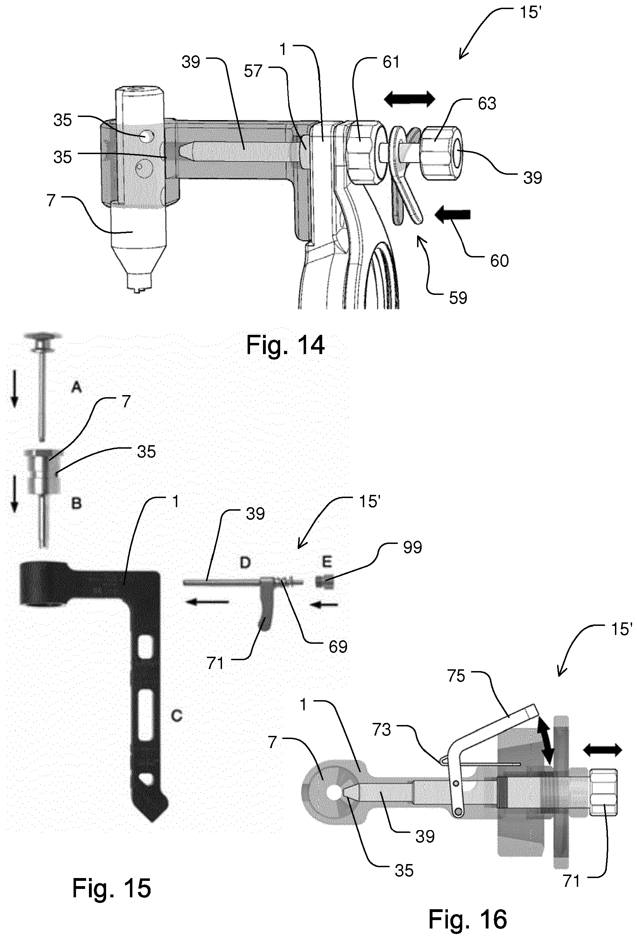

Der erste Abschnitt ist mit einem zweiten Abschnitt (in Fig. 13 der rechte Abschnitt) des Führungsbogens 1 verbunden. Der zweite Abschnitt umfasst die Zielvorrichtung 5. Die Verbindung beider Abschnitte kann beispielsweise mittels einer Buchse 57 ausgeführt sein. In der Buchse 57 wird der Einraststift 39 (oder Bolzen der Einrastanordnung) geführt, der in Fig. 13 von dem Führungsbogen 1 verdeckt ist und in Fig. 14 als weitere Einrastanordnung 15' näher erläutert wird. Der Einraststift 39 wird vorzugsweise mittels wenigstens eines Hebels 59 oder einer anders gestalteten Anordnung betätigt oder bewegt. Der Hebel 59 ist in Fig. 13 in zwei verschiedenen Positionen dargestellt. Die Funktion des Hebels 59 wird zu Fig. 14 näher beschrieben.

Der zweite Abschnitt des Führungsbogens 1 umfasst die Verstellvorrichtung 3 sowie die Zielvorrichtung 5, die in Fig. 13 in Explosionsdarstellung, d. h. in nicht montiertem Zustand, dargestellt sind. Im montierten Zustand wird die Verstellvorrichtung 3 am oberen Ende (bezogen auf die Ansicht in Fig. 13) beispielsweise mit der Buchse 57 verbunden, wie dies in Fig. 14 gezeigt ist.

Die Verstellvorrichtung 3 wird, beispielsweise am unteren Ende hiervon, mittels einer Verbindungsschraube 65 mit dem zweiten Abschnitt des Führungsbogens 1 verbunden oder daran fixiert.

Die Zielvorrichtung 5 wird mittels eines sogenannten Drehspanners 67 zwischen der Verstellvorrichtung 3 und dem zweiten Abschnitt des Führungsbogens 1 fixiert.

Bzgl. der Funktion der Zielvorrichtung 5 wird auf die Beschreibung der Fig. 10 verwiesen.

**Fig. 14** zeigt die weitere Einrastanordnung 15' der Ausführungsform der Fig. 13.

Die Einrastanordnung 15' basiert auf einer magnetisch unterstützten Fixierung des Einraststifts 39 in den Einrastpositionen 35. Die magnetische Kopplung wird durch einen Magneten oder magnetischen Werkstoff, oder wie hier durch zwei Magnete 61, 63 realisiert, die am äußeren Ende des Einraststifts 39 angeordnet sein können und die, einander zugewandt, unterschiedliche Polungen aufweisen. Der erste Magnet 61 kann beispielsweise mit der Buchse 57 und/oder mit dem Führungsbogen 1 verbunden sein. Der zweite Magnet 63 kann mit dem äußeren Ende des Einraststifts 39 verbunden sein. Mittels des Hebels 59 können die beiden Magnete auseinandergedrückt werden und somit den Einraststift 39 aus der Einrastposition 35 entkoppeln oder herausziehen. Dazu wird der Hebel 59 in Pfeilrichtung 60 nach innen gedrückt, so dass aufgrund des abgewinkelten Hebels 59 der Einraststift 39 und der Magnet 63 nach außen geschoben werden.

**Fig. 15** zeigt eine weitere Einrastanordnung 15' mit dem Einraststift 39, einem Handstück 71, einer Spiralfeder 69 und einer Schraube 99. Zur Montage werden zunächst die Teile A und B in Pfeilrichtung in den Führungsbogen 1 eingeführt. Danach werden die Teile D und E (Einraststift 39, Handstück 71, Spiralfeder 69) in Pfeilrichtung in den Führungsbogen 1 eingeführt. Anschließend wird die Schraube 99 (Teil E) in den Führungsbogen 1 eingeschraubt und damit der Einraststift 39, das Handstück 71 und die Spiralfeder 69 im Führungsbogen 1 fixiert. Das Handstück 71 wurde in diesem Montageschritt durch den innen hohlen Führungsbogen 1 im Winkelbereich hindurchgeschoben, und ragt anschließend aus dem Führungsbogen 1 im Winkelbereich heraus. In diesem eingerasteten Zustand ist die Spiralfeder 69 komprimiert und vorgespannt. Zum Entkoppeln des eingerasteten Einraststifts 39 aus der Einrastposition 35 in der Hülse 7 wird der Einraststift 39 mittels des Handstücks 71 durch manuelles Hineindrücken des Handstücks 71 in den Führungsbogen 1 nach außen gezogen, der Einraststift 39 aus einer Einrastposition 35 herausgezogen und die Spiralfeder 69 weiter komprimiert. Nach einer erneuten Positionierung des Führungsbogens 1 kann der Einraststift 39 mittels Zurückführens des Handstücks 71 nach innen und eines Entspannens der Spiralfeder 69 in deren Ausgangszustand wieder in eine der Einrastpositionen 35 der Hülse 7 eingerastet werden.

**Fig. 16** zeigt eine weitere Einrastanordnung15' mit einer Blattfeder 73 und einem Hebel 75. Analog der Beschreibung der Fig. 15 wird die Blattfeder 73, und/oder aufgrund des Spannens, mittels Herunterdrücken des Hebels 75 gespannt. Gleichzeitig mit dem Spannen der Blattfeder 73 wird der Einraststift 39 mittels des Handgriffs 71 aus der Einrastposition 35 herausgezogen und entkoppelt. Nach einer neuen Positionierung des Führungsbogens 1 kann der Einraststift 39 wieder in eine weitere Einrastposition 35 in der Hülse 7 eingeführt und eingerastet werden.

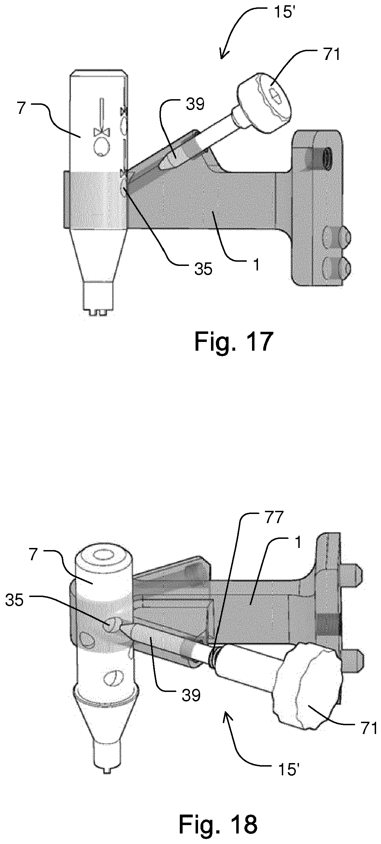

**Fig. 17** zeigt eine weitere Einrastanordnung 15' mit einem Einraststift 39 (der Einraststift 39 kann als Einsteckbolzen bezeichnet werden), der von schräg oben in eine Einrastposition 35 in der Hülse 7 eingeführt wird.

**Fig. 18** zeigt eine weitere Einrastanordnung 15' mit einem Einraststift 39 zu dessen seitlichen Einführen in eine Einrastposition 35 der Hülse 7. Der Einraststift 39 kann nach dem Einrasten mittels Betätigen oder Drehen des Handgriffs 71 und einem optionalen Gewinde 77 fixiert werden.

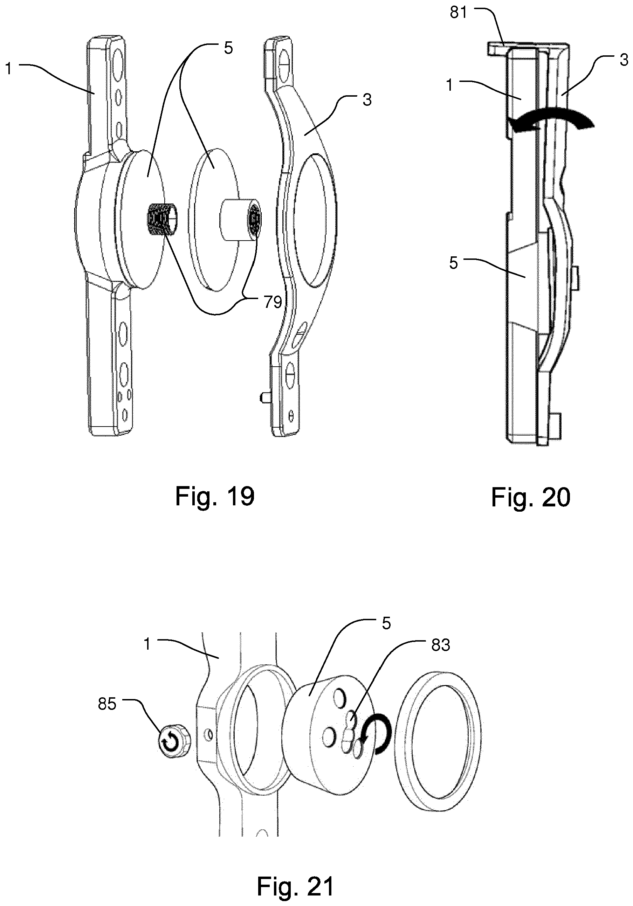

**Fig. 19** zeigt eine zweigeteilte Zielvorrichtung 5 der Ausführungsform aus Fig. 13. Zwei Abschnitte der Zielvorrichtung 5 können beispielsweise mittels eines Gewindes 79 miteinander verschraubt werden. Das Außengewinde des in Fig. 19 linken Abschnitts der zweigeteilten Zielvorrichtung 5 wird in das Innengewinde des in Fig. 19 rechten Abschnitts der Zielvorrichtung 5 eingeschraubt.

Mittels einer zweigeteilten Zielvorrichtung 5 können verschiedene Vorteile erzielt werden, wie beispielsweise eine Kombination verschiedener Materialien (höhere Festigkeiten einer Buchse zur Führung eines Instruments zum Einsetzen der Verriegelungsvorrichtung oder der Verriegelungsschraube 23, 23', siehe z. B. Fig. 2) oder einer Erhöhung der Festigkeit oder Biegesteifigkeit der Zielvorrichtung 5.

**Fig. 20** zeigt eine Verstellvorrichtung 3 mit einer Schnappvorrichtung 81 zum Fixieren der Verstellvorrichtung 3 an dem Führungsbogen 1 in einer Seitenansicht. Die Pfeilrichtung zeigt die Richtung des Zuklappens, Verschließens oder Einschnappens der Verstellvorrichtung 3 auf oder am Führungsbogen 1. Die Schnappvorrichtung 81 wird beim Einschnappvorgang insbesondere elastisch verformt oder verbogen, so dass sich nach dem Einschnappen die Schnappvorrichtung 81 am Führungsbogen 1 einhakt. Zum Öffnen kann die Schnappvorrichtung 81 elastisch hochgebogen und aufgeklappt werden.

Im geöffneten Zustand kann beispielsweise die Zielvorrichtung 5 ausgetauscht oder adaptiert werden.

**Fig. 21** zeigt eine weitere Zielvorrichtung 5 mit einer Löcher-Anordnung 83 für ein Instrument 23 (siehe Fig. 22). Gegenüber den Ausführungsformen der Zieleinrichtung 5 beispielsweise der Fig. 11 oder Fig. 12, in denen die Zielvorrichtung 5 nur ein Loch aufweist, die Zielvorrichtung 5 jedoch zwischen dem Führungsbogen 1 und der Verstellvorrichtung 3 dreidimensional in mehreren Achsrichtungen beweglich ist, ist die Zielvorrichtung 5 in Fig. 21 nur um eine Achse (die Mittelachse der zylindrischen Zielvorrichtung 5) drehbar. Diese Einschränkung der Bewegung kann vorteilhaft sein, um beispielsweise die Winkel für ein Instrument 23 in den jeweiligen Löchern zu begrenzen und somit die Winkel zum Setzen von Verriegelungsschrauben 21 mittels der Positioniereinrichtung 100 einzugrenzen.

Die Zielvorrichtung 5 kann mittels einer Feststellschraube 85 fixiert werden.

**Fig. 22** zeigt die Positioniervorrichtung 100' der Fig. 10 mit dem Führungsbogen 1, der Verstellvorrichtung 3, der Hülse 7, dem Marknagel 19, der Zielvorrichtung 5 sowie dem Instrument 23 zum Einsetzen der Verriegelungsschraube 21 in einer weiteren Ansicht. Die in Fig. 10 dargestellten ringförmig angeordneten Nuten sind zur vereinfachten Darstellung in Fig. 22 nicht gezeigt.

Die Verstellvorrichtung 3 ist mittels (beispielsweise) vier (optional) punktförmiger Fixierungen 87 mit dem Führungsbogen 1 verbunden.

**Fig. 23** zeigt die Positioniervorrichtung 100' der Fig. 22 in einer Schnittdarstellung.

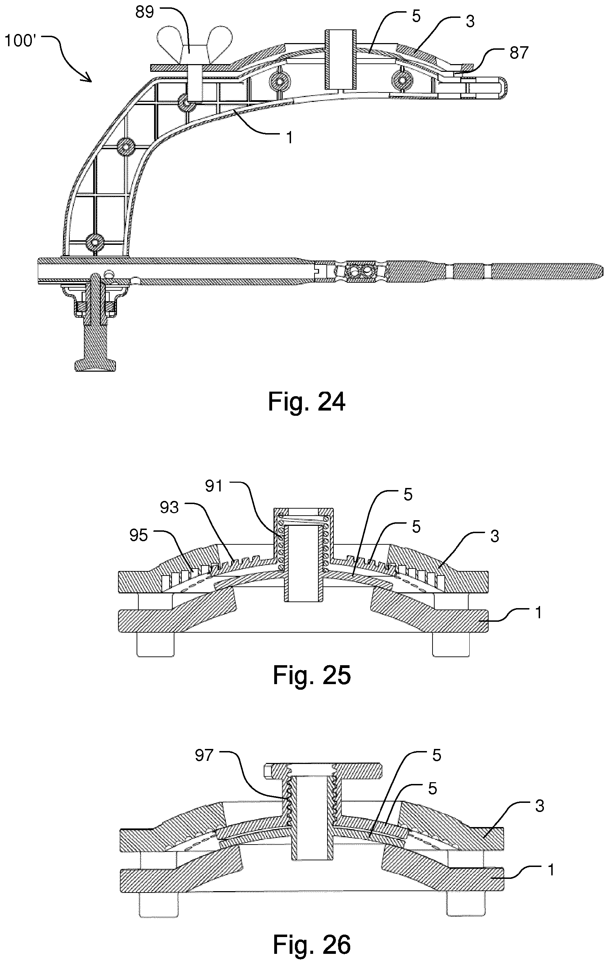

**Fig. 24** zeigt die Positioniervorrichtung 100' der Fig. 22 in einer Schnittdarstellung mit einem Klappen-Konzept zum Fixieren der Verstellvorrichtung 3 auf dem Führungsbogen 1. Das Klappen-Konzept weist in diesem Ausführungsbeispiel nur zwei (oder optional nur eine) punktförmige Fixierungen 87 auf einer Seite der Verstellvorrichtung 3 auf. Die Fixierung 87 kann beispielsweise als Gelenk ausgeführt sein. Auf der gegenüberliegenden Seite wird die Verstellvorrichtung 3 mittels einer Flügelschraube 89 (oder einem Exzenter, einem Schnapphaken oder ähnlichem) mit dem Führungsbogen 1 verbunden. Mittels dieser Verbindung wird die Verstellvorrichtung 3 auf den Führungsbogen 1 gedrückt oder gepresst, so dass die Zielvorrichtung 5 zwischen der Verstellvorrichtung 3 und dem Führungsbogen 1 festgeklemmt oder fixiert wird.

Mittels des Klappen-Konzepts ist es beispielsweise vorteilhaft möglich, die Zielvorrichtung 5 auszutauschen.

**Fig. 25** zeigt eine Zielvorrichtung 5, welche mittels eines Federn-Konzepts zwischen der Verstellvorrichtung 3 und dem Führungsbogen 1 angeordnet und bewegbar ist. Die Zielvorrichtung 5 umfasst in dieser Ausführungsform zwei Abschnitte oder Schalen, zwischen denen eine Spiralfeder 91 angeordnet ist. Mittels der Spiralfeder 91 wird der obere Abschnitt (bezogen auf Fig. 25) nach oben gedrückt. Dadurch rasten die auf der Oberseite angeordneten Erhebungen 93 in Bohrungen 95 (oder Nuten) auf der Unterseite der Verstellvorrichtung 3 ein. Beispielsweise können jeweils zehn Erhebungen 93 und Bohrungen 95 auf der Oberseite bzw. Unterseite angeordnet sein.

**Fig. 26** zeigt eine weitere Zielvorrichtung 5, welche mittels eines Gewinde-Konzepts zwischen der Verstellvorrichtung 3 und dem Führungsbogen 1 angeordnet und bewegbar ist. Mittels eines Gewindes 97 können die obere Schale der Zielvorrichtung 5 gegen die Unterseite der Verstellvorrichtung 3 und die Unterseite der unteren Schale der Zielvorrichtung 5 gegen die Oberseite des Führungsbogens 1 gedrückt und fixiert werden.

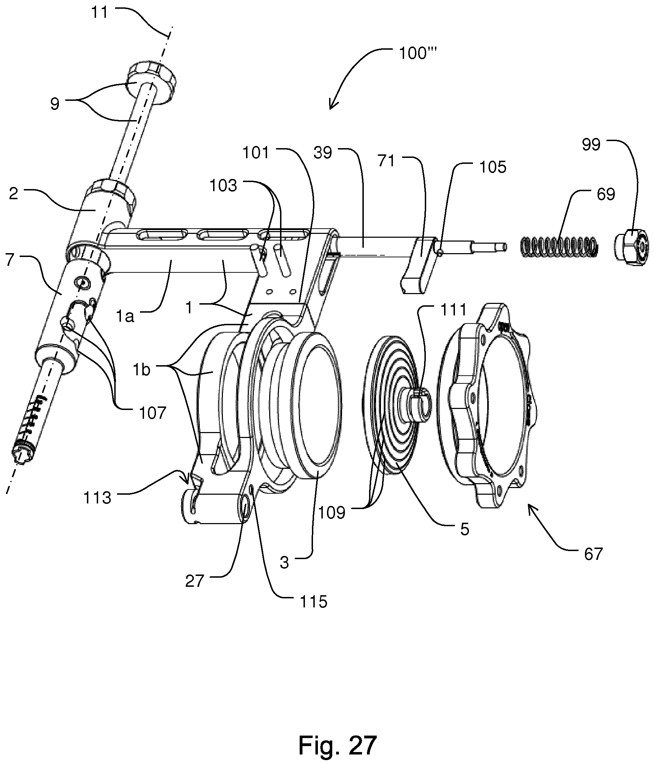

**Fig. 27** zeigt eine weitere Ausführungsform der erfindungsgemäßen Positioniervorrichtung 100"'.

Der Führungsbogen 1 ist zweigeteilt ausgeführt. Ein erster Abschnitt 1a (in Fig. 27 oben) ist mit der Hülsenführung 2, insbesondere wieder lösbar, verbunden. Alternativ kann die Verbindung eine nicht wieder lösbare stoffschlüssige Verbindung sein, z. B. eine Lötverbindung, eine Schweißverbindung oder eine Klebverbindung. Die stoffschlüssige, integrale Verbindung kann ein einstückiges Bauteil sein, z. B. aus einem Material mittels Guß und/oder spanender Bearbeitung hergestellt. Der erste Bereich 1a kann mittels eines generativen Herstellungsverfahrens, z. B. mittels eines Lasersinterverfahrens oder eines Rapid Prototyping Verfahrens hergestellt sein.

Ein zweiter Abschnitt 1b des Führungsbogens 1 kann einteilig oder mehrteilig hergestellt sein.

Der erste Bereich 1a und der zweite Abschnitt 1b können mittels einer formschlüssigen und/oder einer stoffschlüssigen Verbindung an einer Schnittstelle 101 miteinander verbunden sein. Beispielsweise kann der zweite Abschnitt 1b mittels eines Absatzes in einen hohlen Endabschnitt des ersten Abschnitts 1a formschlüssig hineingeschoben werden. Anschließend kann diese formschlüssige Verbindung mittels eines oder mehrerer Bolzen 103 (die Bolzen 103 können Passstifte sein) fixiert und gesichert werden. Diese formschlüssige Verbindung kann zusätzlich mittels einer Klebung gesichert werden. Eine Klebung kann vorteilhaft sein, um eine spielfreie Verbindung auch nach längerem Gebrauch und mehrfachen mechanischen Belastungen zu sichern. Eine spielfreie Verbindung kann für eine exakte Positionierung von Marknägeln mittels der erfindungsgemäßen Positioniervorrichtung 100"' für einen Therapieerfolg wichtig sein.

Die Montage des Einraststifts 39, des Handstücks 71, der Spiralfeder 69 und der Schraube 99 mit dem Führungsbogen 1 erfolgt analog der Beschreibung zu Fig. 13. Zusätzlich ist in der Ausführungsform in Fig. 27 ein kleiner Stift 105 (der Stift 105 kann als Pin bezeichnet werden) an dem Einraststift 39 befestigt oder verbunden. Die Spiralfeder 69 und der Stift 105 sind derart ausgebildet, dass die Spiralfeder 69 in der Regel zunächst mechanisch elastisch verformt wird, um über den Stift 105 auf den Einraststift 39 geschoben werden zu können. (in Fig. 27 wird die Spiralfeder 69 nach links über den Stift 105 in Richtung des Handstücks 71 geschoben). Damit kann sich die Spiralfeder 69 ohne erneute elastische Verformung nicht selbstständig von dem Einraststift 39 lösen. Dies hat den Vorteil, dass eine Sterilisation der Anordnung ohne eine Demontage der Spiralfeder 69 erfolgen kann.

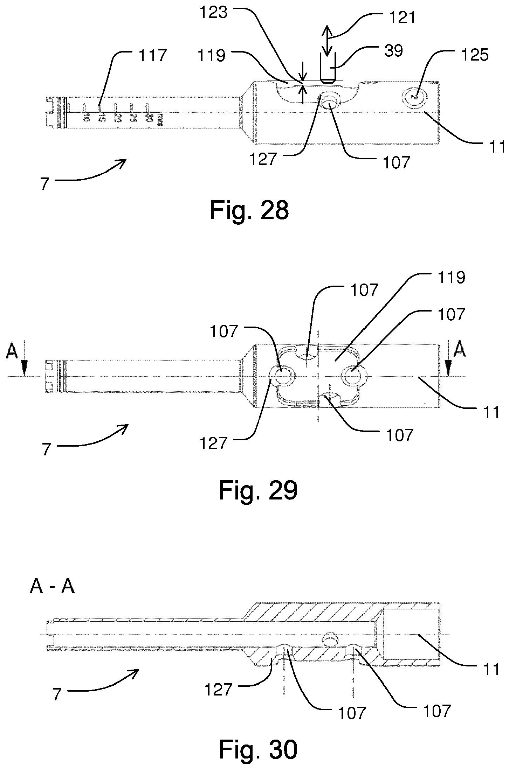

Die Hülse 7 der Ausführungsform in Fig. 27 wird zu den Fig. 28 bis Fig. 30 näher erläutert. Die Anordnung in Fig. 27 stellt einen teilmontierten Zustand dar. Zur weiteren Montage wird die Hülsenführung 2 über die Hülse 7 geschoben. Anschließend kann der Einraststift 39 in eine der Bohrungen 107 hineingeschoben werden. Die Führungsvorrichtung 9 wird zur weiteren Montage entlang der Längsachse 11 der Hülse 7 nach unten (bezogen auf Fig. 27) geschoben, um den Marknagel 19 (s. Fig. 13) wieder lösbar mit der Führungsvorrichtung 9 zu verbinden.

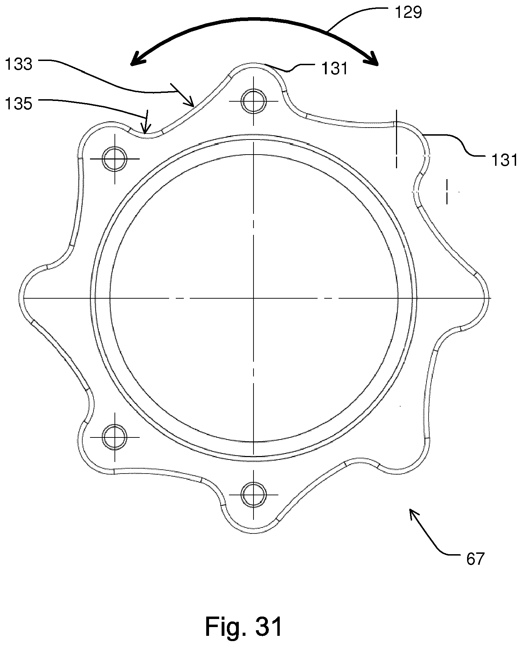

Der Drehspanner 67 verbindet und fixiert im montierten Zustand die Zielvorrichtung 5 und die Verstellvorrichtung 3 mit dem Führungsbogen 1. Der Drehspanner 67 wird zur Fig. 31 näher erläutert.

Die Zielvorrichtung 5 weist auf wenigstens einer Oberfläche (in Fig. 27 auf der rechten Seite) konzentrische Ringe 109 auf. Diese rein optischen Ringe dienen dem Benutzer der Orientierung der aktuellen Positionierung der Zielvorrichtung 5, die verschiebbar zwischen dem Drehspanner 67 und der Verstellvorrichtung 3 angeordnet ist.

Weiterhin weist die Zielvorrichtung 5 auf dem Umfang des zentrischen, hülsenförmigen Ansatzes einen Längsschlitz 111 auf. Dieser Längsschlitz 111 dient der elastischen Verformung des ringförmigen Ansatzes bei einem Einführen eines Instruments zum Einsetzen der Verriegelungsschraube 23 (s. Fig. 11). Im nicht verformten Zustand ist der Innendurchmesser des Ansatzes geringfügig kleiner als der Durchmesser des Instruments 23. Der Ansatz wird bei dem Einführen des Instruments 23 elastisch verformt und aufgeweitet, und kann anschließend aktiv, mittels Kraftaufwand, gegen den Reibwiderstand zwischen Ansatz und Instrument verschoben oder gedreht werden. Aufgrund des Reibwiderstands kann das Instrument nur aktiv bewegt werden, nicht jedoch herausfallen. Diese Art der Klemmung ist vorteilhaft, wenn das Instrument 23 nicht ständig manuell fixiert und gehalten werden kann, aber trotzdem in einer vorgegebenen Position verbleiben soll.

Der zweite Abschnitt 1b des Führungsbogens 1 weist eine Positionierhilfe 27 als Bohrung für weitere Instrumente 23' (s. Fig. 11) zum Einsetzen von Verriegelungsschrauben auf. Diese Positionierhilfe 27 weist an einem Ende (in Fig. 27 links) ebenfalls einen Längsschlitz 113 (verdeckt) auf, der dieselbe Funktion wie der Längsschlitz 111 hat. Somit ist ein Instrument 23', welches in die Bohrung der Positionierhilfe 27 hineingeschoben wird, einerseits bewegbar, wird jedoch andererseits aufgrund des Reibwiderstands geklemmt, um ein Herausfallen zu verhindern.

Weiterhin weist der zweite Abschnitt 1b eine Bohrung 115 für einen Verbindungsstift, insbesondere für einen Passstift, auf. Mittels des Paßstifts kann der zweite Abschnitt 1b insbesondere mit einer Verlängerung (in Fig. 27 nicht dargestellt) verbunden werden, um beispielsweise weitere Bohrungen für Positionierhilfen zum Einsetzen von weiteren Verriegelungsschrauben in den Marknagel vorzusehen.

Die in Fig. 27 dargestellten Bauteile können aus einem oder verschiedenen Materialien hergestellt sein. Vorzugsweise sind die Bauteile zweiter Abschnitt 1b, Verstellvorrichtung 3, Zielvorrichtung 5 und Drehspanner 67 aus einem Kunststoff hergestellt, die übrigen Bauteile aus einem oder verschiedenen metallischen Werkstoffen. Rein exemplarisch können die Bauteile aus Kunststoff aus einem oder verschiedenen der folgenden Kunststoffe hergestellt sein oder diese aufweisen: PEEK (Polyetheretherketon); PEEK faserverstärkt; PEEK faserverstärkt in unterschiedlichen Konzentrationen der Fasern; Polyoxymethylen (POM); kohlenstofffaserverstärkter Kunststoff (CFK); Polyarylsulfon, insbesondere Polyphenylsulfon (PPSU). Rein exemplarisch sind die Bauteile aus Metall aus einem Edelstahl hergestellt oder weisen dieses auf. Der Edelstahl kann gehärtet und/oder gestrahlt sein.

**Fig. 28** zeigt die Hülse 7 aus Fig. 27 als Einzelteilansicht. Die Längsachse 11 der Hülse entspricht der Längsachse des Marknagels 19, der am linken Ende (bezogen auf Fig. 28) mittels einer Führungsvorrichtung 9 (s. Fig. 13) adaptiert und fixiert werden kann. Zur Orientierung beispielsweise der Eindringtiefe des Marknagels ist auf der Hülse eine Markierung mit Millimeterangaben aufgeprägt.

Die Hülse 7 weist gegenüber der Ausführungsform der Hülse 7 in den Fig. 3 bis Fig. 7 keine Kulissenführung 33 auf.

**Fig. 29** zeigt die Hülse 7 aus Fig. 28 in einer um 90 Grad um die Längsachse 11 gedrehten Ansicht.

**Fig. 30** zeigt die Hülse 7 in einer Halbschnittdarstellung A - A entsprechend der in Fig. 29 gezeigten Schnittebene A - A.

Entsprechend der bereits weiter oben diskutierten Funktionsweise der Positioniervorrichtung 100 und dem Führungsbogen 1, z. B. zu den Fig. 10 und Fig. 15, wird mittels eines Einraststifts 39 der Führungsbogen 1 relativ zur Hülse 7 positioniert.

Die Positionierung erfolgt hier beispielhaft dadurch, dass der Einraststift 39 in unterschiedlichen Bohrungen 107 in der Hülse 7 positioniert werden kann und mittels dieser Positionierung eine Verriegelungsschraube 21 mittels eines Instruments 23 und einer Zielvorrichtung 5 im Marknagel 19 und im umgebenden Röhrenknochen fixiert wird.

Der Anwender der erfindungsgemäßen Positioniervorrichtung 100 kann dabei zwischen vorgegebenen Bohrungen 107 auswählen, welche Verriegelungsschraube 21 er jeweils im Marknagel 19 und im Röhrenknochen positionieren und fixieren möchte. Bei dieser Auswahl soll der Einraststift 39 beispielhaft jedoch nur zwischen den vorgegebenen Bohrungen 107 bewegt werden können, um eine schnelle und zielgenaue Fixierung zu ermöglichen. Zur Erreichung dieses Ziels werden die Verschiebemöglichkeiten des Einraststifts 39 durch einen, beispielsweise ausgefrästen, Bereich 119 eingeschränkt. Die Verschieberichtung des Einraststifts 39 entlang seiner Längsachse, in Fig. 28 durch den Pfeil 121 dargestellt, wird optional, beispielsweise durch Anschläge, entsprechend begrenzt, so dass der Einraststift 39 vorzugsweise nicht über den äußeren Durchmesser der Hülse 7 hinaus zurückgezogen werden kann. Die Verschiebemöglichkeiten innerhalb des Bereichs 119 ist in Fig. 28 durch den Spalt 123 verdeutlicht und dargestellt.

Im montierten Zustand der Positioniervorrichtung 100 sind die Bohrungen 107 durch die Hülsenführung 2 des Führungsbogens 1 optional verdeckt. Um dem Anwender eine Orientierung zur momentanen Position des Einraststifts 39 zu geben, sind auf der Hülse 7 im nicht verdeckten Bereich vorzugsweise Markierungen 125, beispielsweise Nummerierungen, auf der Oberfläche eingeprägt oder angebracht. Die Markierungen korrespondieren jeweils zu den zugehörigen Bohrungen 107.

Der Bereich 119 in der Ausführungsform der Fig. 28 bis Fig. 30 ist rein exemplarisch gewählt. Er kann beispielsweise enger und kleiner gewählt werden, um die Führungsmöglichkeiten des Einraststifts 39 einzuschränken und damit eine schnellere und präzisere Positionierung in einer Bohrung 107 zu ermöglichen oder auszuwählen.

Die Bohrungen 107 sind optional mit Anschrägphasen versehen, um das Einführen des Einraststifts 39 in die Bohrungen 107 zu erleichtern. Die Bohrungen 107 sind vorzugsweise mit Passungen versehen, um eine spielfreie Positionierung zu ermöglichen.

**Fig. 31** zeigt einen weiteren Drehspanner 67. Der Drehspanner 67 weist in dieser Ausführungsform unsymmetrische Eingriffskonturen oder Umfangs- oder Außenkonturen zum, insbesondere manuellen, Festziehen und Lösen auf.

Die optionale, unsymmetrische Eingriffskontur der Fig. 31 kann als Sägezahnkontur bezeichnet werden. Der Drehspanner 67 wird beispielsweise im Uhrzeigersinn (bezogen auf die Aufsicht in Fig. 31) der Drehrichtung 129 festgezogen und entgegen dem Uhrzeigersinn gelöst. Die Flanke im Uhrzeigersinn ist für diese Bedienung deutlich flacher als die Flanke zum Lösen. Damit kann zum Festziehen nur ein geringes Drehmoment aufgebracht werden. Wird das Drehmoment zu stark erhöht, rutschen die Hand oder die Finger im Eingriff bei einem manuellen Festziehen durch den Nutzer über die Noppen 131 hinaus. Damit kann vorteilhaft erreicht werden, dass keine zu hohen Drehmomente zum Festziehen des Drehspanners aufgebracht werden können. Sehr hohe Drehmomente könnten eine Beschädigung oder ein Bruch dieses, vorzugsweise in Kunststoff, hergestellten Bauteils verursachen. Vorzugsweise ist es bei der vorliegenden Ausgestaltung einfacher, Drehmoment zum Lösen als Drehmoment zum Festziehen auf den Drehspanner aufzubringen. Der Nutzer kann sich daher sicher sein, den von ihm manuell festgezogenen Drehspanner mit eigener Kraft auch wieder per Hand lösen zu können.

Alternativ zu einer rein manuellen Betätigung des Drehspanners 67 kann weiterhin ein Werkzeug benutzt werden.

Die unterschiedlichen Steigungen der Flanken werden mittels der Radien 133 und 135 definiert. Rein exemplarisch kann der Radius 133 ca. 6 mm und der Radius 135 ca. 49 mm betragen.

### Bezugszeichenliste

- 100, 100', 100", 100"': Positioniervorrichtung
- x: x-Richtung; Verschieberichtung der Verstellvorrichtung entlang des Führungsbogens
- y: y-Richtung; Richtung senkrecht zur Verschieberichtung der Verstellvorrichtung entlang des Führungsbogens; in Umfangsrichtung der Hülse
- 1: Führungsbogen
- 1a: erster Bereich oder Abschnitt des Führungsbogens
- 1b: zweiter Bereich oder Abschnitt des Führungsbogens
- 2: Hülsenführung
- 3: Verstellvorrichtung
- 5: Zielvorrichtung
- 7: Hülse
- 9: Führungsvorrichtung
- 11: Längsachse der Hülse
- 13: Umfangsrichtung der Hülse
- 15, 15': Einrastanordnung
- 17: Feststellschraube
- 19: Marknagel
- 21, 21': Verriegelungsvorrichtung; Verriegelungsschraube
- 23, 23': Instrument zum Einsetzen der Verriegelungsvorrichtung oder der Verriegelungsschraube
- 25: Durchgangsöffnung
- 27: Positionierhilfe
- 28: Innengewinde
- 29: Kulissenführung
- 31: Führungsabsatz
- 33: nutförmige Erweiterung der Kulissenführung in Umfangsrichtung der Hülse
- 35: Einrastposition; Langlöcher in der Hülse
- 37: Markierung zur Positionskontrolle
- 39: Einraststift; Bolzen der Einrastanordnung
- 41: Blattfeder
- 43: Bolzenanordnung
- 45: Hebel
- 47: Zahnradverbindung
- 49: Steg der Hülse
- 51: Gewinde
- 53: Stift; in Radialrichtung federnd gelagert
- 55: Werkzeug
- 57: Buchse
- 59: Hebel
- 60: Verschieberichtung des Hebels; Pfeilrichtung
- 61: Magnet
- 63: Magnet
- 65: Verbindungsschraube
- 67: Drehspanner
- 69: Spiralfeder für Einraststift
- 71: Handstück
- 73: Blattfeder
- 75: Hebel
- 77: Gewinde am Einraststift
- 79: Gewinde der zweigeteilten Zielvorrichtung
- 81: Schnappvorrichtung
- 83: Löcher-Anordnung
- 85: Feststellschraube
- 87: punktförmige Fixierung
- 89: Flügelschraube
- 91: Spiralfeder für Zielvorrichtung
- 93: Erhebungen auf der Oberseite der Zielvorrichtung
- 95: Bohrungen auf der Unterseite der Verstellvorrichtung
- 97: Gewinde für Zielvorrichtung
- 99: Schraube
- 101: Schnittstelle zwischen erstem und zweitem Abschnitt des Führungsbogens
- 103: Bolzen; Paßstift
- 105: Stift; Pin
- 107: Bohrungen der Hülse
- 109: konzentrische Ringe der Zielvorrichtung
- 111: Längsschlitz des hülsenförmigen Ansatzes der Zielvorrichtung
- 113: Längsschlitz der Positionierhilfe
- 115: Bohrung für Paßstift
- 117: Markierung mit Millimeterangaben
- 119: Verschiebebereich durch Einraststift
- 121: Verschieberichtung des Einraststifts
- 123: Spaltbreite zum Verschieben des Einraststifts
- 125: Markierungen
- 127: Anschrägphase
- 129: Drehrichtungen des Drehspanners
- 131: Noppen
- 133: erster Radius des Drehspanners
- 135: zweiter Radius des Drehspanners

## Patentansprüche

1. Positioniervorrichtung (100, 100', 100", 100"') zum Positionieren und/oder Fixieren eines Marknagels (19) in einem Röhrenknochen, umfassend
- einen Führungsbogen (1), welcher aufweist:
- eine Verstellvorrichtung (3), wobei die Verstellvorrichtung (3) wenigstens eine Zielvorrichtung (5) aufweist, und wobei die Zielvorrichtung (5) ausgebildet ist, um eine Verriegelungsvorrichtung (21) oder ein Instrument (23) zum Einwirken auf die Verriegelungsvorrichtung (21) zumindest abschnittsweise aufzunehmen, und wobei die Verstellvorrichtung (3) gemeinsam mit der im Gebrauch der Positioniervorrichtung (100, 100', 100", 100"') auf den Marknagel (19) auszurichtenden Zielvorrichtung (5) längs (x-Richtung) des Führungsbogens (1) verschiebbar angeordnet ist; und
- eine Hülsenführung (2) mit einer Hülse (7), wobei die Hülse (7) relativ zur Hülsenführung (2) dreh- und verschiebbar in der Hülsenführung (2) angeordnet ist, und wobei die Hülse (7) eine durchgängige Längsöffnung zum Aufnehmen einer Führungsvorrichtung (9) hierin oder hierdurch aufweist.

2. Positioniervorrichtung (100, 100', 100", 100"') nach Anspruch 1, ferner eine in die Hülse (7) eingesteckte Führungsvorrichtung (9) aufweisend.

3. Positioniervorrichtung (100, 100', 100", 100"') nach Anspruch 1 oder 2, wobei die Hülse (7) und/oder die Führungsvorrichtung (9) einen Verbindungsabschnitt zu ihrem lösbaren Verbinden mit einem Marknagel (19) aufweist.

4. Positioniervorrichtung (100, 100', 100", 100"') nach einem der vorangegangenen Ansprüche, wobei die Hülse (7) eine Kulissenführung (29) zum Führen eines Abschnitts, insbesondere eines Führungsabsatzes (31), des Führungsbogens (1) oder der Hülsenführung (2) entlang der Hülse (7), oder umgekehrt, aufweist.

5. Positioniervorrichtung (100, 100', 100", 100"') nach einem der vorangegangenen Ansprüche, wobei der Führungsbogen (1) einen zwischen wenigstens zwei Positionen bewegbaren Einraststift (39) aufweist, und wobei die Hülse (7) wenigstens eine Einrastposition (35) zum Einstecken oder Einrasten des Einraststifts (39) hierin aufweist.

6. Positioniervorrichtung (100, 100', 100", 100"') nach Anspruch 4 oder 5, wobei die Einrastposition (35) eine in Umfangsrichtung (13) der Hülse (7) ausgerichtete Längsnut (33) aufweist, wobei die Längsnut (33) ein Verschieben des Einraststifts (39) in Umfangsrichtung (13) der Hülse (7) im eingerasteten Zustand des Einraststifts (39) in der Einrastposition (35) ermöglicht.

7. Positioniervorrichtung (100, 100', 100", 100"') nach einem der vorangegangenen Ansprüche, wobei die Hülse (7) Markierungen (37) zur Kontrolle der Position und/oder Stellung des Führungsbogens (1) relativ zur Hülse (7) aufweist.

8. Positioniervorrichtung (100, 100', 100", 100"') nach einem der vorangegangenen Ansprüche, wobei die Führungsvorrichtung (9) ein Außengewinde (51) aufweist, wobei die Führungsvorrichtung (9) zum Führen eines Werkzeugs (55) in ihrem Inneren hohl, mit einer Längsöffnung und/oder rohrförmig ausgestaltet ist.

9. Positioniervorrichtung (100, 100', 100", 100"') nach einem der vorangegangenen Ansprüche, wobei der Führungsbogen (1) zumindest in Abschnitten als Kreisbogen ausgebildet ist, und wobei die Verstellvorrichtung (3) angeordnet ist, um zumindest in Abschnitten längs des Kreisbogens verschiebbar zu sein.

10. Positioniervorrichtung (100, 100', 100", 100"') nach einem der vorangegangenen Ansprüche, wobei die Zielvorrichtung (5) eine auf den Marknagel (19) gerichtete, die Verstellvorrichtung (3) durchstoßende Öffnung (25) aufweist.

11. Positioniervorrichtung (100, 100', 100", 100"') nach einem der vorangegangenen Ansprüche, wobei der Führungsbogen (1) Anschläge zur Begrenzung des Verschiebewegs der Verstellvorrichtung (3) längs des Kreisbogens aufweist.

12. Positioniervorrichtung (100, 100', 100", 100"') nach einem der vorangegangenen Ansprüche, wobei die Zielvorrichtung (5) in Längsrichtung (x-Richtung) des Führungsbogens (1) und senkrecht (y-Richtung) zur Längsrichtung (x-Richtung) des Führungsbogens (1) verschiebbar oder bewegbar ist.

13. Positioniervorrichtung (100, 100', 100", 100"') nach einem der vorangegangenen Ansprüche, wobei die Zielvorrichtung (5) in der Verstellvorrichtung (3) und relativ zu dieser verschiebbar angeordnet ist.

14. Positioniervorrichtung (100, 100', 100", 100"') nach Anspruch 13, wobei die Zielvorrichtung (5) in wenigstens einer ersten Position zum Bewegen der Zielvorrichtung (5) relativ zur Verstellvorrichtung (3) beweglich angeordnet ist und in wenigstens einer zweiten Position zum wieder lösbaren Fixieren der Zielvorrichtung (5) relativ zur Verstellvorrichtung (3) unbeweglich angeordnet ist.

## Claims

1. A positioning device (100, 100', 100", 100"') for positioning and/or fixing an intramedullary nail (19) in a tubular bone, comprising:
- a guiding bow (1), which comprises:
- an adjusting device (3), wherein the adjusting device (3) comprises at least one targeting device (5), and wherein the targeting device (5) is designed to receive, at least partially, an interlocking device (21) or an instrument (23) for acting on the interlocking device (21), and wherein the adjusting device (3) is arranged to be displaceable along (x-Richtung) the guiding bow (1), together with the targeting device (5) intended to be aligned with the intramedullary nail (19) when using the positioning apparatus (100, 100', 100", 100"') ; and
- a sleeve guide (2) having a sleeve (7), the sleeve (7) being arranged to be rotatable and displaceable in the sleeve guide (2) relative to the sleeve guide (2), and the sleeve (7) comprising a longitudinal through-opening for receiving a guiding device (9) therein or therethrough.

2. The positioning device (100, 100', 100", 100"') according to claim 1, further comprising a guiding device (9) inserted into the sleeve (7).

3. The positioning device (100, 100', 100", 100"') according to claim 1 or 2, wherein the sleeve (7) and/or the guiding device (9) comprise/s a connecting portion for its releasable connection with an intramedullary nail (19).

4. The positioning device (100, 100', 100", 100'") according to anyone of the preceding claims, wherein the sleeve (7) comprises a sliding block guide (29) for guiding a portion, in particular a guiding portion (31), of the guiding bow (1) or of the sleeve guide (2) along the sleeve (7), or vice versa.

5. The positioning device (100, 100', 100", 100"') according to anyone of the preceding claims, wherein the guiding bow (1) comprises a snap-in pin (39) movable between at least two positions, and wherein the sleeve (7) comprises at least one snap-in position (35) for inserting or snapping the snap-in pin (39) therein.

6. The positioning device (100, 100', 100", 100"') according to claim 4 or 5, wherein the snap-in position (35) comprises a longitudinal groove (33) aligned in the circumferential direction (13) of the sleeve (7),
the longitudinal groove (33) allowing the snap-in pin (39) to move in the circumferential direction (13) of the sleeve (7) when the snap-in pin (39) is in the snap-in position (35).

7. The positioning device (100, 100', 100", 100"') according to anyone of the preceding claims, wherein the sleeve (7) comprises markings (37) for checking the position and/or the location of the guiding bow (1) relative to the sleeve (7).

8. The positioning device (100, 100', 100", 100"') according to anyone of the preceding claims, wherein the guiding device (9) comprises an external thread (51), the guiding device (9) for guiding a tool (55) within its hallow interior being designed with a longitudinal opening and/or being tubular.

9. The positioning device (100, 100', 100", 100"') according to anyone of the preceding claims, wherein the guiding bow (1) is designed, at least partially, as a circular bow, and wherein the adjusting device (3) is arranged so as to be movable, at least partially, along the circular bow.

10. The positioning device (100, 100', 100", 100"') according to anyone of the preceding claims, wherein the targeting device (5) comprises an opening (25) directed towards the intramedullary nail (19) which pierces the adjusting device (3).

11. The positioning device (100, 100', 100", 100"') according to anyone of the preceding claims, wherein the guiding bow (1) comprises stops for limiting the displacement path of the adjusting device (3) along the circular bow.

12. The positioning device (100, 100', 100", 100"') according to anyone of the preceding claims, wherein the targeting device (5) is displaceable or movable in the longitudinal direction (x-Richtung) of the guiding bow (1) and perpendicular (y-Richtung) to the longitudinal direction (x-Richtung) of the guiding bow (1).

13. The positioning device (100, 100', 100", 100'") according to anyone of the preceding claims, wherein the targeting device (5) is displaceably arranged in the adjusting device (3) and is relative thereto.

14. The positioning device (100, 100', 100", 100'") according to claim 13, wherein the targeting device (5) is movably arranged in at least one first position for moving the targeting device (5) relative to the adjusting device (3) and is non movably arranged in at least one second position for releasably fixing the targeting device (5) relative to the adjusting device (3).

## Revendications

1. Un dispositif de positionnement (100, 100', 100", 100"') pour positionner et/ou fixer un clou intramédullaire (19) dans un os tubulaire, comprenant:
- un arceau de guidage (1) qui comprend:
- un dispositif de réglage (3), où le dispositif de réglage (3) comprend au moins un dispositif de visée (5) , et où le dispositif de visée (5) est conçu pour recevoir, au moins partiellement, un dispositif de verrouillage (21) ou un instrument (23) permettant d'agir sur le dispositif de verrouillage (21), et où le dispositif de réglage (3) est agencé de manière à pouvoir se déplacer le long (x-Richtung) de l'arceau de guidage (1), conjointement avec le dispositif de visée (5) devant être aligné avec le clou intramédullaire (19) lors de l'utilisation du dispositif de positionnement (100, 100', 100", 100"').
- un guide de manchon (2) ayant un manchon (7), le manchon (7) étant agencé de manière à pouvoir pivoter et coulisser dans le guide de manchon (2) par rapport au guide de manchon (2), et où le manchon (7) comprend une ouverture longitudinale traversante pour recevoir un dispositif de guidage (9) à l'intérieur ou au travers de celle-ci.

2. Le dispositif de positionnement (100, 100', 100", 100"') selon la première revendication, comprenant en outre un dispositif de guidage (9) inséré dans le manchon (7).

3. Le dispositif de positionnement (100, 100', 100", 100"') selon la revendication 1 ou 2, où le manchon (7) et/ou le dispositif de guidage (9) comprennent/comprend une section de raccordement pour leur/son raccordement amovible à un clou intramédullaire (19).

4. Le dispositif de positionnement (100, 100', 100", 100'") selon l'une quelconque des revendications précédentes, où le manchon (7) comprend un guide à coulisse (29) pour guider une section, notamment une section de guidage (31), de l'arceau de guidage (1) ou du guide de manchon (2) le long du manchon (7), ou vice versa.

5. Le dispositif de positionnement (100, 100', 100", 100'") selon l'une quelconque des revendications précédentes, où l'arceau de guidage (1) comprend une broche d'encliquetage (39) mobile entre au moins deux positions, et où le manchon (7) comprend au moins une position d'encliquetage (35) pour y insérer ou y encliqueter la broche d'encliquetage (39).

6. Le dispositif de positionnement (100, 100', 100", 100"') selon la revendication 4 ou 5, où la position d'encliquetage (35) comprend une rainure longitudinale (33) alignée dans la direction circonférentielle (13) du manchon (7),
la rainure longitudinale (33) permettant un déplacement de la broche d'encliquetage (39) dans la direction circonférentielle (13) du manchon (7) lorsque la broche d'encliquetage (39) est en position d'encliquetage (35).

7. Le dispositif de positionnement (100, 100', 100", 100"') selon l'une quelconque des revendications précédentes, où le manchon (7) comprend des repères (37) permettant de vérifier la position et/ou l'emplacement de l'arceau de guidage (1) par rapport au manchon (7).

8. Le dispositif de positionnement (100, 100', 100", 100"') selon l'une quelconque des revendications précédentes, où le dispositif de guidage (9) comprend un filetage externe (51), le dispositif de guidage (9) pour le guidage d'un outil (55) étant creux à l'intérieur, avec une ouverture longitudinale et/ou étant tubulaire.

9. Le dispositif de positionnement (100, 100', 100", 100"') selon l'une quelconque des revendications précédentes, où l'arceau de guidage (1) est conçu, au moins partiellement, comme un arceau circulaire, et où le dispositif de réglage (3) est agencé de manière à pouvoir coulisser, au moins partiellement, le long de l'arceau circulaire.

10. Le dispositif de positionnement (100, 100', 100", 100"') selon l'une quelconque des revendications précédentes, où le dispositif de visée (5) comprend une ouverture (25) dirigée vers le clou intramédullaire (19) et traversant le dispositif de réglage (3).

11. Le dispositif de positionnement (100, 100', 100", 100"') selon l'une quelconque des revendications précédentes, où l'arceau de guidage (1) comprend des butées pour limiter la trajectoire de déplacement du dispositif de réglage (3) le long de l'arceau circulaire.

12. Le dispositif de positionnement (100, 100', 100", 100"') selon l'une quelconque des revendications précédentes, où le dispositif de visée (5) est déplaçable ou mobile dans une direction longitudinale (x-Richtung) de l'arceau de guidage (1) et perpendiculairement (y-Richtung) à la direction longitudinale (x-Richtung) de l'arceau de guidage (1).

13. Le dispositif de positionnement (100, 100', 100", 100'") selon l'une quelconque des revendications précédentes, où le dispositif de visée (5) est agencé de façon mobile dans le dispositif de réglage (3) et par rapport à celui-ci.

14. Le dispositif de positionnement (100, 100', 100", 100"') selon la revendication 13, où le dispositif de visée (5) est agencé de manière mobile dans au moins une première position permettant le déplacement du dispositif de visée (5) par rapport au dispositif de réglage (3), et est agencé de manière non mobile dans au moins une seconde position permettant de fixer le dispositif de visée (5) de manière amovible par rapport au dispositif de réglage (3).
